# EUROPEAN PATENT SPECIFICATION

(11) **EP 4 178 455 B1**
(45) Date of publication and mention of the grant of the patent: **11.11.2026**
(21) Application number: 20944595.6
(22) Date of filing: 23.12.2020
(51) Int. Cl.: A61B 17/02, A61B 17/072, A61B 17/00

(54) **POWERED HANDLE ASSEMBLY FOR SURGICAL DEVICES**
ANGETRIEBENE GRIFFANORDNUNG FÜR CHIRURGISCHE VORRICHTUNGEN
ENSEMBLE POIGNÉE MOTORISÉ POUR DISPOSITIFS MÉDICAUX

(30) Priority: 09.07.2020 WO PCT/CN2020/100951
(43) Date of publication of application: 17.05.2023
(73) Proprietor: Covidien LP, Mansfield, MA 02048-1113 (US)
(72) Inventor: LI, Shouwei, Shanghai 201114 (CN); ZHANG, Zhihua, Shanghai 201114 (CN)
(74) Representative: Zimmermann & Partner Patentanwälte mbB
(86) International application number: PCT/CN2020/138521
(87) International publication number: WO 2022/007344

(56) References cited:
- EP-A1- 3 682 817
- CN-A- 103 989 497
- CN-A- 107 028 633
- CN-A- 107 257 664
- CN-A- 109 350 160
- CN-A- 109 350 160
- CN-A- 110 785 128
- US-A1- 2014 263 564
- US-A1- 2017 224 334

## Description

### FIELD

This technology is generally related to handle assemblies for surgical devices and, more particularly, to a handle assembly for a powered surgical stapling device.

### BACKGROUND

Surgical devices are commonly used during surgical procedures to perform a variety of different surgical operations including stapling, grasping, cutting, and sealing tissue to name a few. Typically, a surgical device includes a handle assembly that a clinician, e.g., a surgeon, grasps to actuate the surgical device. Certain types of surgical devices perform multiple tasks and have different capabilities to allow the device to more easily access tissue within a body cavity. For example, powered surgical stapling devices include actuation buttons for approximating jaws of a tool assembly of the stapling device, for applying staples to tissue, and for cutting tissue. These stapling devices can also include knobs to facilitate articulation and/or rotation of the tool assembly in relation to the handle assembly.

Endoscopic surgical devices are used during minimally invasive surgical procedures to minimize trauma inflicted on a patient during the surgical procedure. Typically, endoscopic surgical devices include a handle assembly, an elongate body, and a tool assembly that is supported on a distal portion of the elongate body. In order to better access tissue within a body cavity during a surgical procedure, the tool assembly can be pivotably mounted to the elongate body and movable between a non-articulated position in which the tool assembly is aligned with the elongate body to articulated positions in which the tool assembly defines acute angles with the elongate body.

In order to pivot the tool assembly between the articulated and non-articulated positions, the handle assembly includes an articulation mechanism that is coupled to an articulation link positioned within the elongate body. The articulation link is movable longitudinally within the elongate body in response to actuation of the articulation mechanism to pivot the tool assembly within the body cavity.

A continuing need exists in the surgical arts for a powered handle assembly including an articulation mechanism for pivoting a tool assembly between non-articulated and articulated positions. US Patent Application US 2017/0224334 describes a surgical instrument with end effector and includes an articulation system including an articulation driver laterally offset and a transversely extending cross link. US Patent Application US 2014/0263564 describes an interchangeable shaft assembly for use with a surgical instrument which includes a frame that supports a plurality of drive systems. Chinese Patent Application CN 109350160 describes a surgical device capable of being operated with a single hand and rotating in multiple directions.

### SUMMARY

The invention is defined by the appended claims.

In one aspect is provided a handle assembly for a surgical device comprising:
a body supporting at least one clutch switch ;
a drive assembly supported within the housing, the drive assembly including a drive screw, a screw nut, and a drive rod, the drive rod having a proximal portion and a distal portion, the screw nut defining a threaded bore and including external splines, the drive screw extending through the threaded bore, and the drive rod having a proximal portion coupled to the drive screw;
an articulation mechanism supported within the housing, the articulation mechanism including a first articulation gear, a second articulation gear, and an articulation screw, the first articulation gear defining a through bore and including splines positioned within the through bore and outer gear teeth, the second articulation gear including outer gear teeth that are engaged with the outer gear teeth of the first articulation gear;
a clutch supported within the housing between the first articulation gear and the screw nut, the clutch movable between a clamp/fire position in which the clutch is engaged with the external splines of the screw nut and an articulation position in which the clutch is engaged with the splines of the first articulation gear;
a biasing member supported within the housing, the biasing member urging the clutch towards the clamp/fire position; and
a motor coupled to the clutch, the motor operable to rotate the clutch within the housing to actuate one of the drive assembly or the articulation mechanism.

The handle assembly may further include a first bevel gear coupled to the motor and a second bevel gear coupled to the first bevel gear.

The second bevel gear may define a through bore and a longitudinal slot that communicates with the through bore, the through bore of the second bevel gear receiving the screw nut and the drive screw, and the clutch including a raised extension that is received within the longitudinal slot to rotatably couple the clutch to the second bevel gear.

The articulation mechanism may include an articulation link that is coupled to the articulation screw such that movement of the articulation screw causes longitudinal movement of the articulation link.

The at least one clutch switch may include a clutch switch on each side of the body, the clutch switches coupled to the clutch by forked members, and the clutch switches movable along the body to move the clutch from the clamp/fire position to the articulation position.

The body may support guide rods and the forked members define openings, the guide rods extending through the openings in the forked members to guide movement of the forked members as the clutch is moved from the clamp/fire position to the articulation position.

The handle assembly may further including a safety toggle mechanism supported on the body, the safety toggle mechanism including at least one toggle member and a shaft coupled to the at least one toggle member, the shaft being rotatable in response to manipulation of the at least one toggle member to move the safety toggle mechanism from an inactive position in which the handle assembly is deactivated to an active position in which the handle assembly is activated; and wherein body of the handle assembly supports a contact and the shaft of the safety toggle mechanism includes an arm, wherein in the inactive position of the safety toggle mechanism, the arm is spaced from the contact and the contact is in an open position, and in the active position of the safety toggle mechanism, the arm is engaged with the contact and the contact is in a closed position.

The safety toggle mechanism may include a slider mounted on the shaft, the slider including a stop member; and wherein the drive assembly includes a coupling member that couples the drive screw to the drive rod, the drive screw, the coupling member and the drive rod movable between retracted and advanced positions within the body in response to actuation of the motor when the clutch is in the clamp/fire position.

The coupling member may be positioned to obstruct movement of the safety toggle mechanism from the inactive position to the active position when the coupling member is in its retracted position; and wherein body supports a tapered cam member having a tapered cam surface and proximal stop surface, and the slider includes a projection, the projection movable along the tapered cam surface to move the slider from a first position on the shaft in which the slider is aligned with the tapered cam member to a second position on the shaft in which the slider is positioned outwardly of the tapered cam member as the safety toggle mechanism is moved from the inactive position to the active position, wherein the projection is aligned with the proximal stop surface when the safety toggle mechanism is in the active position to retain the safety toggle mechanism in the active position.

The slider may be urged towards the first position by a biasing mechanism.

In a further aspect is provided a surgical device comprising:
a handle assembly as above and
an adapter assembly defining a longitudinal axis and having a proximal portion and a distal portion, the adapter assembly including an articulation rod, the proximal portion of the adapter assembly coupled to the handle assembly, the articulation rod having a proximal portion coupled to the articulation screw and a distal portion, the drive rod extending through the adapter assembly; and
a tool assembly pivotably coupled to the distal portion of the adapter assembly about an axis that is transverse to the longitudinal axis of the adapter assembly, the distal portion of the articulation rod coupled to the tool assembly to pivot the tool assembly between a non-articulated position in which the tool assembly is aligned with the longitudinal axis and articulated positions in which the tool assembly is misaligned with the longitudinal axis.

The at least one clutch switch may include a clutch switch supported on each side of the body of the handle assembly, the clutch switches coupled to the clutch and movable along the body of the handle assembly to move the clutch from the clamp/fire position to the articulation position; preferably further including a safety toggle mechanism supported on the body, the safety toggle mechanism including at least one toggle member and a shaft coupled to the at least one toggle member, the shaft rotatable in response to manipulation of the at least one toggle member to move the safety toggle mechanism from an inactive position in which the handle assembly is deactivated to an active position in which the handle assembly is activated.

The body of the handle assembly may support a contact and the shaft of the safety toggle mechanism includes an arm, wherein in the inactive position of the safety toggle mechanism, the arm is spaced from the contact and the contact is in an open position, and in the active position of the safety toggle mechanism, the arm is engaged with the contact and the contact is in a closed position; preferably wherein the safety toggle mechanism includes a slider mounted on the shaft, the slider including a stop member.

The drive assembly may include a coupling member that couples the drive screw to the drive rod, the drive screw, the coupling member and the drive rod movable between retracted and advanced positions within the body in response to actuation of the motor when the clutch is in the clamp/fire position; and wherein the coupling member is positioned to obstruct movement of the safety toggle mechanism from the inactive position to the active position when the coupling member is in its retracted position.

Other features of the disclosure will be appreciated from the following description.

### BRIEF DESCRIPTION OF DRAWINGS

Various aspects of a surgical device including a handle assembly according to aspects of the disclosure are described herein below with reference to the drawings, wherein:
FIG. 1 is a side perspective view from a distal end of the disclosed powered surgical device with a tool assembly in a non-articulated position;
FIG. 2 is a side perspective view of a handle assembly of the surgical device shown in FIG. 1 with a housing half-section removed and a clutch in an articulation position;
FIG. 3 is a side perspective view of the handle assembly shown in FIG. 2 with the other housing half-section removed and the clutch in the articulation position;
FIG. 4 is a side perspective view of the handle assembly shown in FIG. 2 with the housing half-section removed and the clutch in the articulation position;
FIG. 5 is a side perspective, exploded view of the handle assembly shown in FIG. 2;
FIG. 6 is an enlarged view of the indicated area of detail shown in FIG. 5;
FIG. 7 is a cross-sectional view taken along section line 7-7 of FIG. 1;
FIG. 8 is a cross-sectional view taken along section line 8-8 of FIG. 7;
FIG. 9 is a cross-sectional view of the handle assembly with the clutch in the articulation position as the articulation mechanism is actuated;
FIG. 10 is a side perspective view of the articulation mechanism and clutch of the handle assembly of the surgical device shown in FIG. 1 with the clutch in the articulation position;
FIG. 11 is a side perspective view of a bevel gear and clutch of the handle assembly shown in FIG. 10 with the parts separated;
FIG. 12 is a side perspective view of the bevel gear and clutch shown in FIG. 11 with the parts assembled;
FIG. 13 is a side perspective view of an articulation gear and the clutch of the handle assembly shown in FIG. 10 with the parts separated;
FIG. 14 is a side perspective view of the tool assembly of the surgical stapling device shown in FIG. 1 with the tool assembly in an articulated position;
FIG. 15 is a side cross-sectional view of the handle assembly shown in FIG. 8 with the clutch in the clamp/fire position and the handle assembly actuated to move a drive rod of the handle assembly proximally;
FIG. 16 is a cross-sectional view of the handle assembly shown in FIG. 15 with the clutch in the clamp/fire position and the handle assembly actuated to move the drive rod of the handle assembly proximally;
FIG. 17 is a side perspective view of the drive assembly and clutch of the handle assembly shown in FIG. 15 with the clutch in the clamp/fire position and the handle assembly actuated to move the drive rod of the handle assembly proximally;
FIG. 18 is a side perspective view of the clutch and screw nut of the drive assembly shown in FIG. 17 with the parts separated;
FIG. 19 is a side cross-sectional view of the handle assembly shown in FIG. 15 with the clutch in the clamp/fire position and the handle assembly actuated to move the drive rod of the handle assembly proximally
FIG. 20 is a side perspective view from a distal end of an alternate version of the disclosed powered surgical device with a tool assembly in a non-articulated position;
FIG. 21 is a side perspective view of the handle assembly of the surgical device shown in FIG. 1 with housing half-sections removed;
FIG. 22 is a side perspective view of the handle assembly shown in FIG. 21 with one of the housing half-sections removed;
FIG. 23 is a side perspective view of the handle assembly shown in FIG. 22 with the housing half-sections and a gear housing;
FIG. 24 is an exploded perspective view of the drive assembly, articulation mechanism, selector switch assembly, and safety toggle mechanism of the handle assembly shown in FIG. 23;
FIG. 25 is a side perspective view from one side of the drive assembly, the articulation mechanism, the safety toggle mechanism, and selector switch assembly of the handle assembly shown in FIG. 24 with the components assembled and clutch switches of the selector switch assembly removed;
FIG. 26 is a side perspective view from the other side of the drive assembly, the articulation mechanism, the safety toggle mechanism, and selector switch assembly of the handle assembly shown in FIG. 24 with the components assembled and clutch switches of the selector switch assembly removed;
FIG. 27 is a perspective view from a distal end of the handle assembly shown in FIG. 21 with the body half-sections and an articulation linkage of an articulation mechanism of the handle assembly removed as the safety toggle mechanism moves towards an activated position;
FIG. 28 is a side perspective view of a gear housing of the handle assembly shown in FIG. 21;
FIG. 29 is a perspective view from the distal end of the handle assembly shown in FIG. 21 with the body half-sections and an articulation linkage of an articulation mechanism of the handle assembly removed with the safety toggle mechanism in the activated position;
FIG. 30 is a perspective view of the handle assembly shown in FIG. 29 with one of the body half-sections and an articulation linkage of an articulation mechanism of the handle assembly removed with the safety toggle mechanism in the activated position;
FIG. 31 is an enlarged view of the indicated area of detail shown in FIG. 29; and
FIG. 32 is a perspective view from above with the body half-sections of the handle assembly shown in FIG. 21 removed and the clutch in an articulation position.

### DETAILED DESCRIPTION

The disclosed surgical device will now be described in detail with reference to the drawings in which like reference numerals designate identical or corresponding elements in each of the several views. However, it is to be understood that the aspects of the disclosure are merely exemplary of the disclosure and may be embodied in various forms. Well-known functions or constructions are not described in detail to avoid obscuring the disclosure in unnecessary detail. Therefore, specific structural and functional details disclosed herein are not to be interpreted as limiting, but merely as a basis for the claims and as a representative basis for teaching one skilled in the art to variously employ the disclosure in virtually any appropriately detailed structure. In addition, directional terms such as front, rear, upper, lower, top, bottom, distal, proximal, and similar terms are used to assist in understanding the description and are not intended to limit the disclosure.

In this description, the term "proximal" is used generally to refer to that portion of the device that is closer to a clinician, while the term "distal" is used generally to refer to that portion of the device that is farther from the clinician. In addition, the term "endoscopic" is used generally to refer to endoscopic, laparoscopic, arthroscopic, and/or any other procedure conducted through a small diameter incision or cannula. Further, the term "clinician" is used generally to refer to medical personnel including doctors, nurses, and support personnel.

This disclosure is directed to a surgical device that includes a powered handle assembly, an adapter assembly extending from the handle assembly, and a tool assembly that is mounted to the adapter assembly about a pivot member. The handle assembly includes a clutch that is movable between an articulation position and a clamp/fire position. In the articulation position, the handle assembly is configured to provide powered articulation of the tool assembly about the pivot member. In the clamp/fire position, the handle assembly is configured to provide actuation of the surgical device.

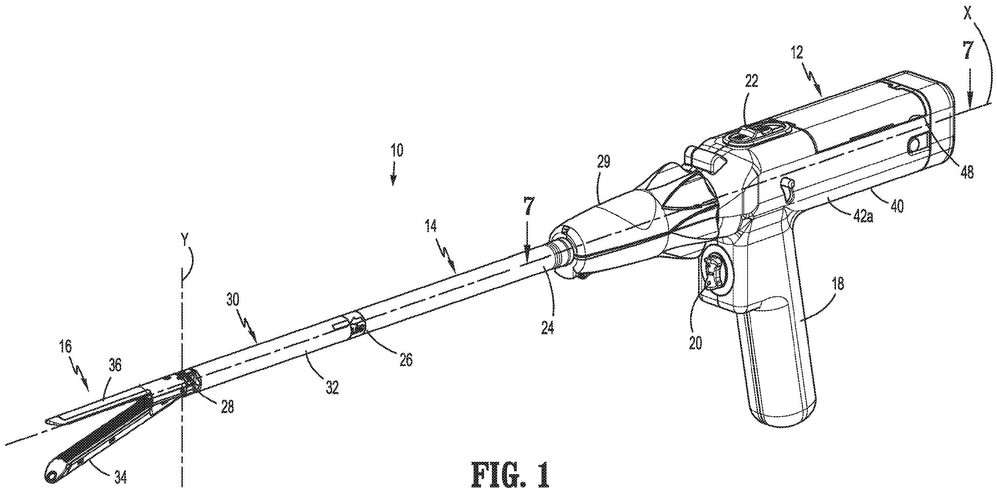

FIG. 1 illustrates a surgical device shown generally as stapling device 10 which includes a handle assembly 12, an elongate body or adapter assembly 14, and a tool assembly 16. The handle assembly 12 includes a stationary handle portion 18, an actuation button 20, and a clutch switch 22. The adapter assembly 14 defines a longitudinal axis "X" and includes a proximal portion 24 that is coupled to the handle assembly 12, and a distal portion 26 that supports the tool assembly 16. The tool assembly 16 is secured to the distal portion 26 of the adapter assembly 14 by a pivot member 28 that defines an axis "Y" that is transverse to the longitudinal axis "X". The tool assembly 16 can articulate about the axis "Y" between an articulated position in which the tool assembly 16 is aligned with the longitudinal axis "Y" and non-articulated positions in which a longitudinal axis of the tool assembly and the longitudinal axis "X" define acute angles. The adapter assembly 14 is supported within a rotation knob 29 that is rotatably coupled to a distal portion of the handle assembly 12. The rotation knob 29 is manually rotatable about the longitudinal axis "X" to rotate the adapter assembly 14 and the tool assembly 16 about the longitudinal axis "X".

In aspects of the disclosure, the tool assembly 16 forms part of a reload assembly 30 that includes a proximal body portion 32 and is releasably coupled to the distal portion of the adapter assembly 14. The reload assembly 30 can be removed from the adapter assembly 14 and replaced to facilitate reuse of the adapter assembly 14 and handle assembly 12. Alternately, the tool assembly 16 can be secured directly to the distal portion 26 of the adapter assembly 14.

As illustrated, the tool assembly 16 can be a stapling device and include a cartridge assembly 34 and an anvil 36 that are movable in relation to each other between an open position and a clamped position. In aspects of the disclosure, the anvil 36 is fixedly secured to the proximal body portion 32 of the reload assembly 30 and the cartridge assembly can pivot between the open and clamped positions. It is envisioned that the cartridge assembly 34 can be fixedly mounted to the proximal body portion 32 and the anvil 36 can pivot between the open and clamped positions. Although the tool assembly 16 is illustrated as a stapling device, it is envisioned that the tool assembly 16 may include a variety of different types of surgical devices including graspers, vessel sealers, clip appliers, stitching devices or the like.

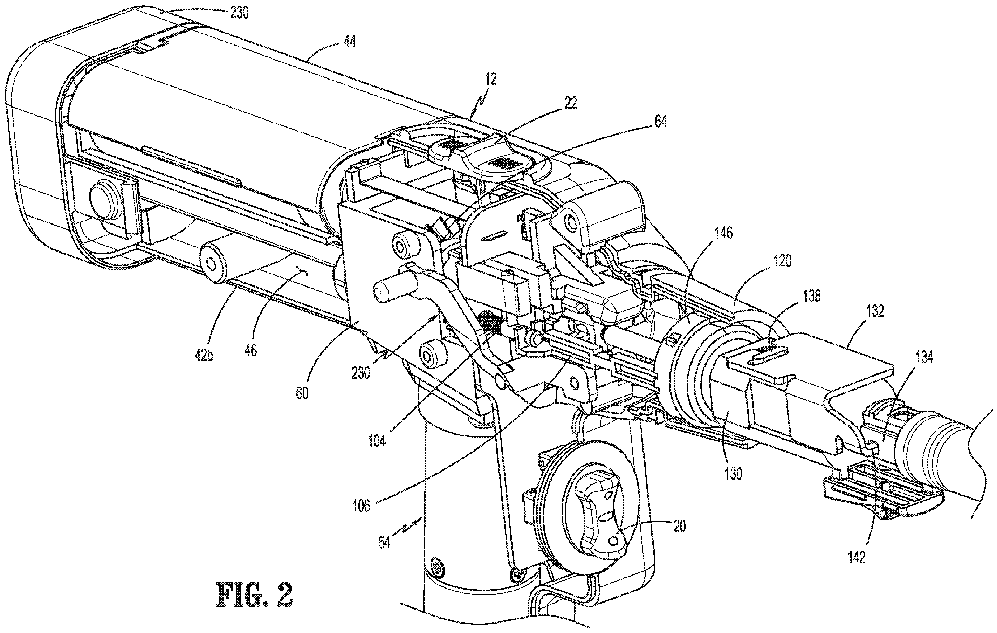

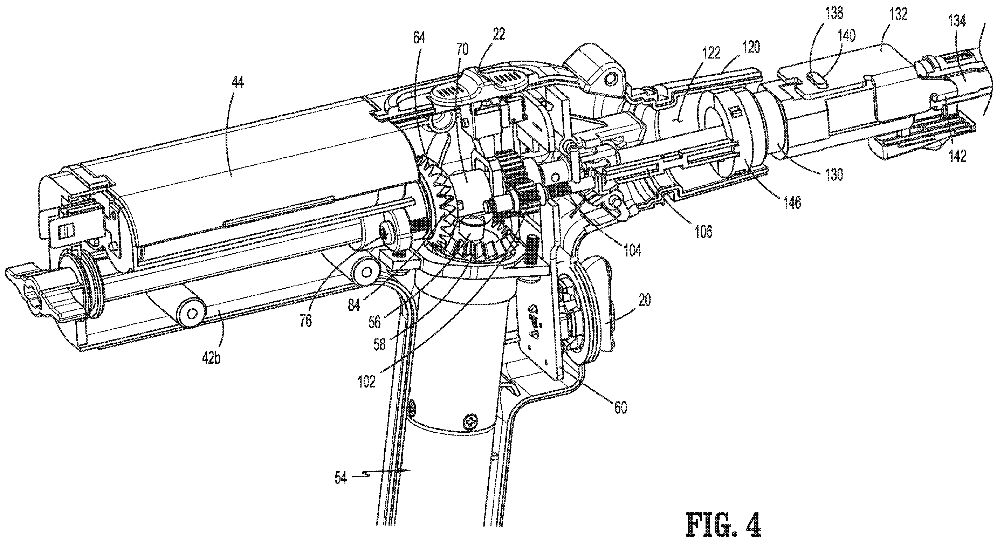

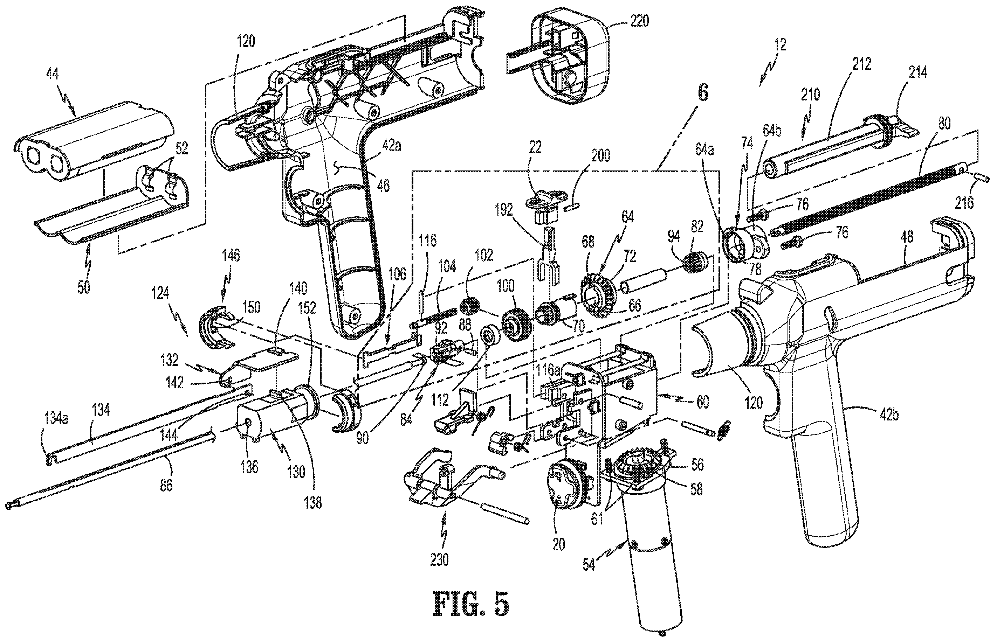

FIGS. 2-5 illustrate the handle assembly 12 of the stapling device 10 which includes a housing 40 (FIG. 1) formed of first and second half-sections 42a and 42b and a battery pack 44. The first and second half-sections 42a and 42b are coupled together to form the stationary handle portion 18 (FIG. 1) and define a cavity 46 having an opening 48 (FIG. 1). The battery pack 44 is received and secured within the opening 48 and includes a base portion 50 (FIG. 5) that includes electrical contacts 52.

The stationary handle portion 18 of the housing 40 of the handle assembly 12 supports a motor 54 that includes a motor shaft 56. The motor shaft 56 is secured to a first bevel gear 58 such that when the motor 54 is energized, the motor shaft 56 rotates the first bevel gear 58. The handle assembly 12 includes a gear housing 60 that is secured above the stationary handle portion 18 of the housing 40 of the handle assembly 12 with, e.g., screws 61 (FIG. 5) such that the first bevel gear 58 is positioned within the gear housing 60. The gear housing 60 supports a printed circuit board 62 ("PCB") which is electrically coupled to the actuation button 20 and to the battery pack 44.

The handle assembly 12 includes a second bevel gear 64 that is engaged with the first bevel gear 58 and is positioned within the gear housing 60. The second bevel gear 64 defines a central through bore 66 (FIG. 5). The central through bore 66 includes longitudinal slots 68 that extend along a length of the central through bore 66. The longitudinal slots 68 are provided to couple the second bevel gear 64 to a clutch 70 as described in further detail below. The second bevel gear 64 is coupled to the first bevel gear 58 such that rotation of the first bevel gear 58 about a first axis causes rotation of the second bevel gear 64 about a second axis that is substantially transverse to the first axis.

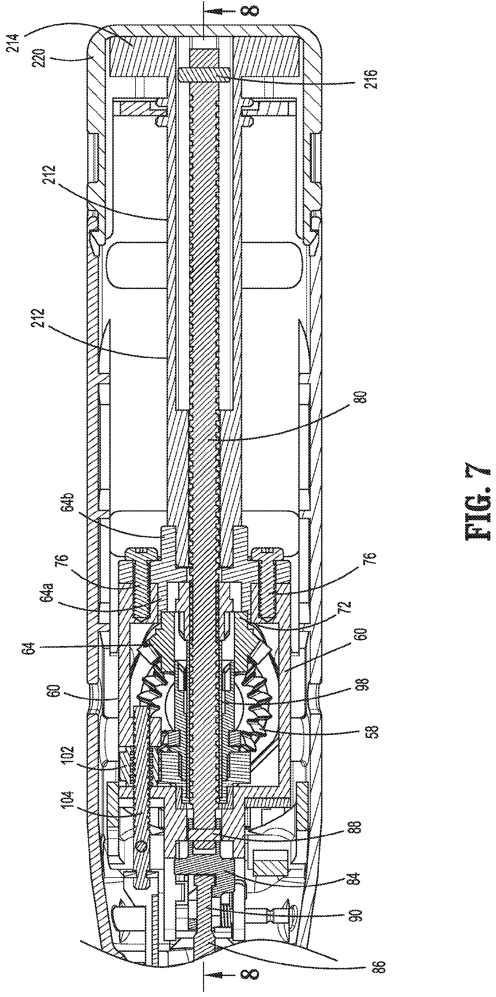

The handle assembly 12 includes a support member 74 that has a distal annular flange 64a (FIG. 7) and a proximal annular flange 64b (FIG. 7). The second bevel gear 64 includes a proximal hub portion 72 that is supported for rotation within the distal annular flange 64a. In aspects of the disclosure, the support member 74 is fixedly secured to the gear housing 60 within the housing 40 of the handle assembly 12 by screws 76 (FIG. 5). The support member 74 defines a through bore 78 that extends between the annular flanges 64a and 64b of the support member 74 (FIG. 7).

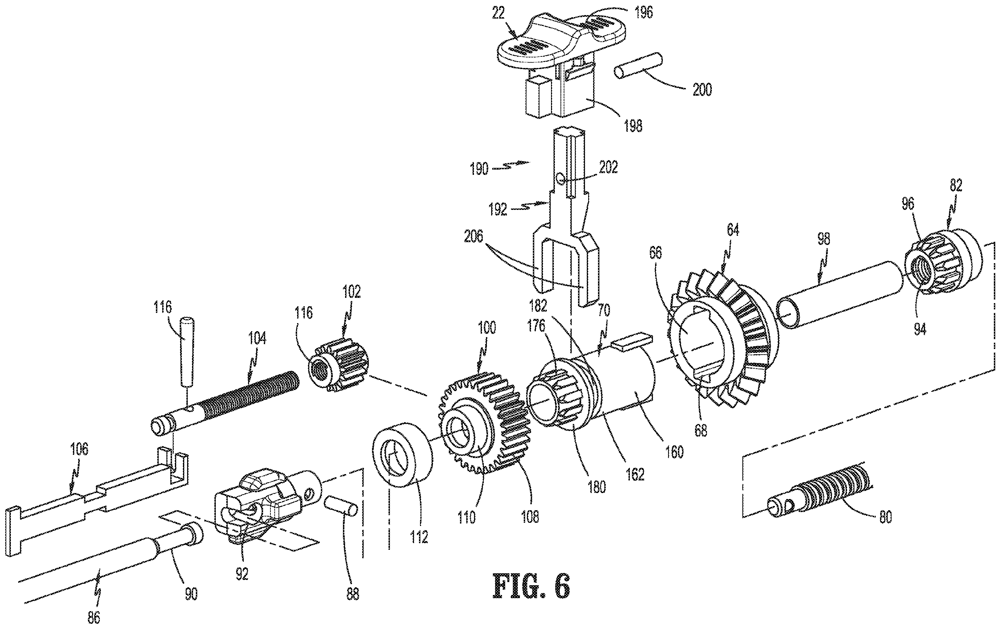

FIGS 5-7 illustrate components of a drive assembly of the handle assembly 12. The drive assembly includes a drive screw 80, a screw nut 82, a coupling member 84, and a drive rod 86. The drive screw 80 extends through the through bore 78 of the support member 74 and has a proximal portion and a distal portion. The distal portion of the drive screw 80 is fixedly coupled to a proximal portion of the coupling member 84 by a pin 88 (FIG. 5). The coupling member 84 includes a distal portion that is coupled to a proximal portion of the drive rod 86. In aspects of the disclosure, the proximal portion of the drive rod 86 includes a stepped portion 90 that is received within a slot 92 (FIG. 6) in the distal portion of the coupling member 84 to axially fix the drive rod 86 to the coupling member 84 but allow the drive rod 86 to rotate in relation to the coupling member 84. The components on the drive assembly are coupled such that the longitudinal movement of the drive screw 80 causes longitudinal movement of the drive rod 86.

The screw nut 82 is received partly within the central through bore 66 of the second bevel gear 64 and within the distal annular flange 64a of the support member 64. The screw nut 82 defines a threaded through bore 94 and includes external gear teeth or splines 96 (FIG. 6). The threaded through bore 94 receives the drive screw 80 to threadedly couple the screw nut 82 about the drive screw 80. The splines 96 engage the clutch 70 when the clutch 70 is in a clamp/fire position as described in further detail below.

A tubular spacer 98 is received about the drive screw 80 and engages a distal portion of the screw nut 82 to maintain the axial position of the screw nut 82 within the housing 40 of the handle assembly 12. The tubular spacer 98 also supports the clutch 70 for movement between the clamp/fire position and an articulation position as described in further detail below.

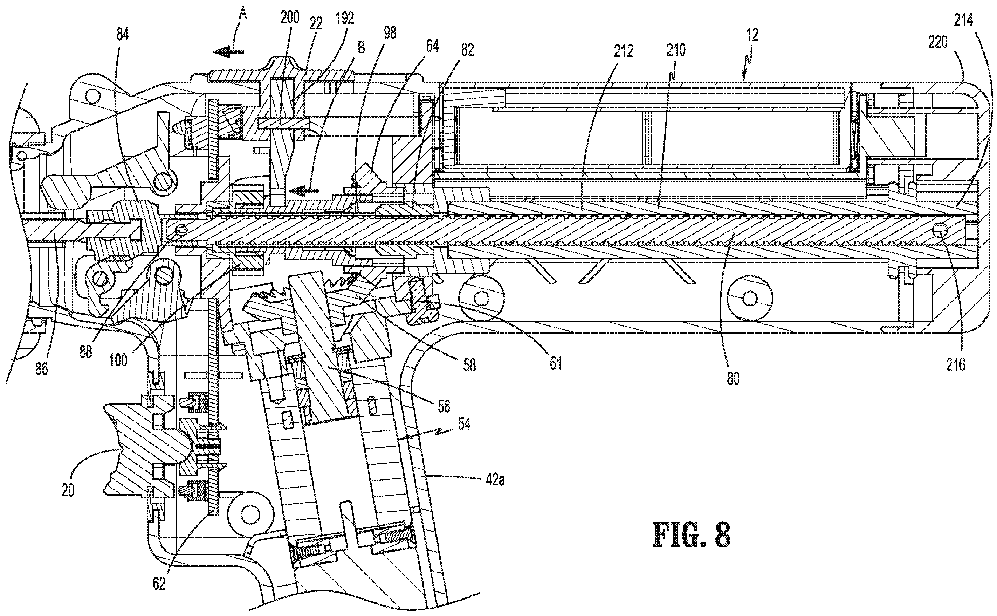

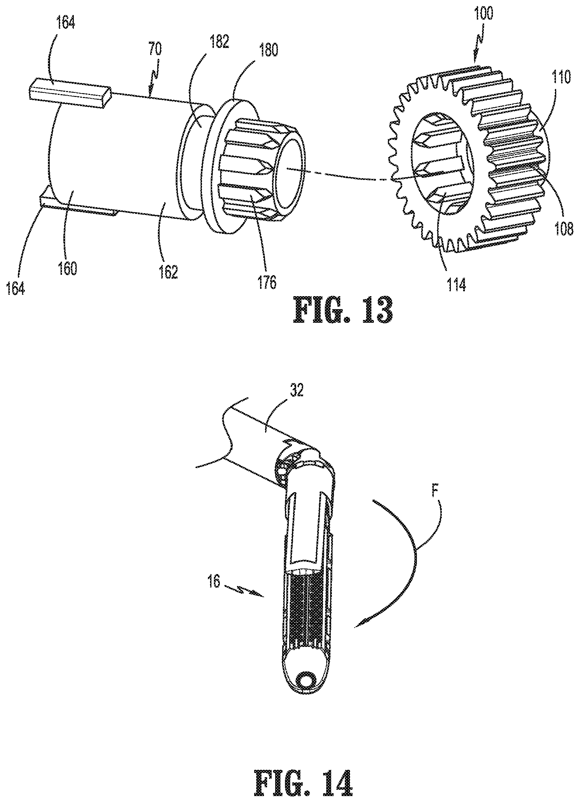

The handle assembly 12 includes an articulation mechanism that includes a first articulation gear 100, a second articulation gear 102, an articulation screw 104, and an articulation link 106. The first articulation gear 100 is supported for rotation on the gear housing 60 and includes outer gear teeth 108. In aspects of the disclosure, the first articulation gear 100 includes a distally extending hub (FIG. 6) that is rotatably supported on a bearing 112 supported on the gear housing 60. The first articulation gear 100 and the bearing 112 define central openings that receive the drive screw 80. The central opening of the first articulation gear 100 is defined by an annular array of splines 114 (FIG. 13) that are engaged by the clutch 70 when the clutch 70 is moved to the articulation position (FIG. 8)

The second articulation gear 102 is rotatably supported on the gear housing 60 and is engaged with the first articulation gear 100. The second articulation gear 102 defines a threaded through bore 116 that receives the articulation screw 104. The second articulation gear 102 is axially fixed to the gear housing 60 such rotation of the second articulation gear 102 causes longitudinal movement of the articulation screw 104. The articulation screw 104 includes a distal portion that is coupled to a proximal portion of the articulation link 106 such that longitudinal movement of the articulation screw 104 causes longitudinal movement of the articulation link 106. In some aspects of the disclosure, the articulation screw 104 includes a guide pin 116 that extends along an axis that is transverse to the longitudinal axis "X" (FIG. 1). The guide pin 116 is received within a channel 116a defined in the gear housing 60 of the handle assembly 12 to prevent the articulation screw 104 from rotating within the housing 40 of the handle assembly 12.

The distal portion of the housing 40 of the handle assembly 12 includes a barrel portion 120 that defines a cavity 122 that receives an articulation linkage 124 (FIG. 5) that includes an articulation body 130, an articulation frame 132, and an articulation rod 134. The articulation body 130 defines a through bore 136 (FIG. 5) that receives the drive rod 86 and includes a protrusion 138. The articulation frame 132 includes body defining an opening 140 that receives the protrusion 138 to fixedly secure the articulation frame 132 to the articulation body 130. The articulation frame 132 includes a finger 142 that is received within an opening 144 (FIG. 5) in a proximal portion of the articulation rod 134 to couple the articulation frame 132 to the articulation rod 134. The articulation rod 134 includes a hooked portion 134a at is distal end that is configured to engage an articulation link (not shown) included in the reload assembly 30 (FIG. 1) to facilitate articulation of the tool assembly 16 upon longitudinal movement of the articulation rod 134. U.S. Patent No. 10,123,799 discloses exemplary aspects of a surgical device including a tool assembly that is mounted for articulation and is adapted to releasably engage an articulation rod of a surgical device.

The articulation linkage 124 also includes a two-part clip 146 that secures the articulation link 106 to the articulation body 130. In aspects of the disclosure, the articulation link 106 has a T-shaped head portion that is received within a T-shaped slot 150 defined in one of the pieces of the two-part clip. The two-part clip 146 is secured about an annular flange 152 formed on a proximal portion of the articulation body 130 to rotatably couple the articulation body 130 to the articulation link 106. This arrangement allows the articulation body 130 to rotate in relation to the articulation link 106, such as when the rotation knob 29 (FIG. 1) is rotated in relation to the handle assembly 12, to facilitate rotation of the adapter assembly 14 and tool assembly 16 about the longitudinal axis "X".

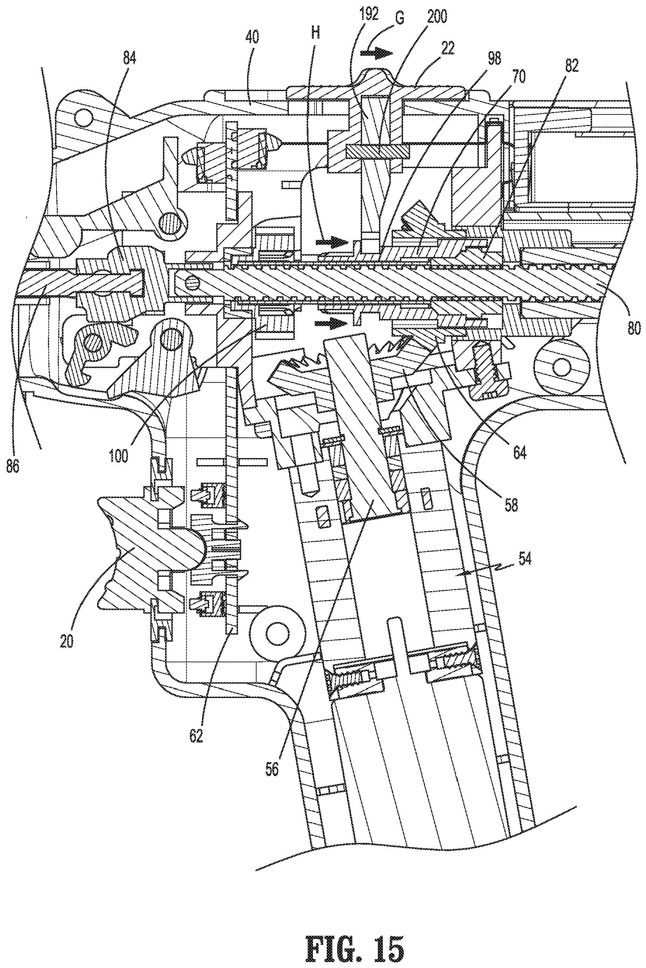

The clutch 70 is slidably positioned about the tubular spacer 98 (FIG. 7) within the through bore 66 of the second bevel gear 64 and is movable between the articulation position (FIG. 8) and the clamp/fire position (FIG. 15). In the articulation position, the clutch 70 is engaged with the first articulation gear 100 and in the clamp/fire position, the clutch 70 is engaged with the screw nut 82.

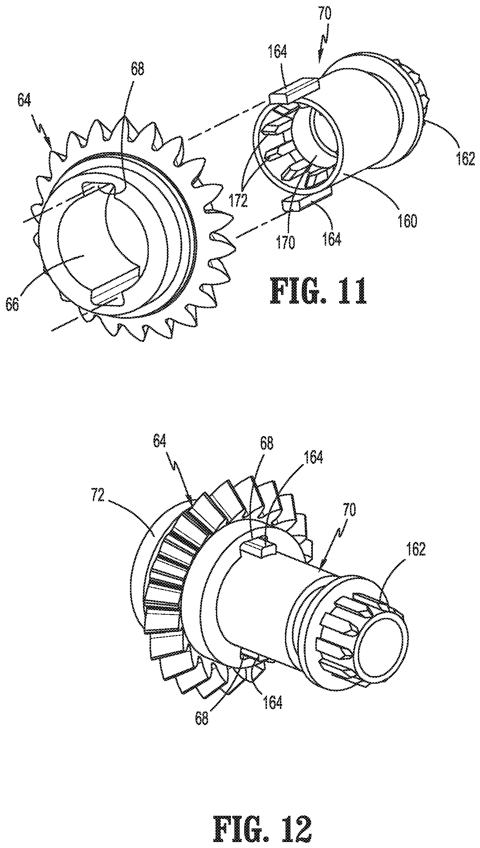

FIGS. 11 and 12 illustrate the clutch 70 and the second bevel gear 64. The clutch 70 includes a proximal portion 160 and a distal portion 162. The proximal portion 160 of the clutch 70 includes raised extensions 164 that extend in a proximal direction towards the screw nut 82 and are received within the longitudinal slots 68 in the second bevel gear 64. Receipt of the raised extensions 164 within the longitudinal slots 68 of the second bevel gear 64 rotatably couples the second bevel gear 64 to the clutch 70. Thus, when the motor 54 (FIG. 5) is energized to rotate the first bevel gear 58 and the second bevel gear 64 as described above, the clutch 70 rotates with the second bevel gear 64.

The clutch 70 defines a through bore 170 and includes internal splines 172. When the clutch 70 is in its clamp/fire position (FIG. 15), the splines 172 on the inner surface of the clutch 70 are engaged with the splines 96 on the screw nut 82. As such, when the motor 54 (FIG. 5) is energized to rotate the clutch 70, the screw nut 82 is also rotated. The distal portion 162 of the clutch 70 includes an annular flange 180 and defines an annular channel 182.

FIG. 13 illustrates the clutch 70 and the first articulation gear 100. The distal portion of the clutch 70 supports a plurality of splines 176. When the clutch 70 is in the articulation position (FIG. 8), the splines 176 on the clutch 70 are engaged with the splines 114 of the first articulation gear 100 such that rotation of the clutch 70 causes rotation of the first articulation gear 100.

FIG. 6 illustrates a selector switch assembly 190 of the handle assembly 12 that is provided to allow a clinician to move the clutch 70 between the articulation and clamp/fire positions. The selector switch assembly 190 includes the clutch switch 22 and a fork 192. The clutch switch 22 includes a finger engagement member 196 and a base portion 198 that extends from the finger engagement member 196 into the housing 40 of the handle assembly 12. The fork 192 is secured to the clutch switch 22 by a pin 200 that extends through the base portion 198 of the clutch switch 22 and is received in an opening 202 formed in the fork 192. The fork 192 includes spaced tines 206 that are received within the annular channel 182 of the clutch 70. When the clutch switch 22 is moved longitudinally along the housing 40 of the handle assembly 12, the clutch 70 is moved longitudinally within the housing 40 along the tubular spacer 98 between the articulation position and the clamp/fire position.

FIGS. 5 and 8 illustrate a manual retraction mechanism 210 of the handle assembly 12 of the surgical stapling device 10 (FIG. 1). The manual retraction mechanism 210 includes tubular body portion 212 and a proximal handle portion 214. The tubular body portion 214 is received about the drive screw 80. The proximal handle portion projects from the proximal portion of the housing 40 of the handle assembly 12 and is rotatably secured to the drive screw 80 by a pin 216 such that rotation of the handle portion 214 of the manual retraction mechanism 210 causes rotation of the drive screw 80 in relation to the screw nut 82 to advance the drive screw 80 within the handle assembly 12. The handle assembly 12 may include a proximal cover 220 to enclose the proximal handle portion 214 of the manual retraction mechanism 210 when not needed. The manual retraction mechanism 210 can be used in the event of failure of the electrical components of the stapling device 10 (FIG. 1) to actuate the tool assembly 216 (FIG. 1), e.g., return the tool assembly 16 to a retracted or unactuated position.

FIG. 5 illustrates a safety switch assembly 230 that is coupled to the PCB 62. The safety switch assembly 230 is provided to prevent inadvertent firing of the stapling device 10 and must be pressed to activate the stapling device 10 (FIG. 1) before the stapling device 10 can be fired. The safety switch assembly 230 will not be described in detail herein.

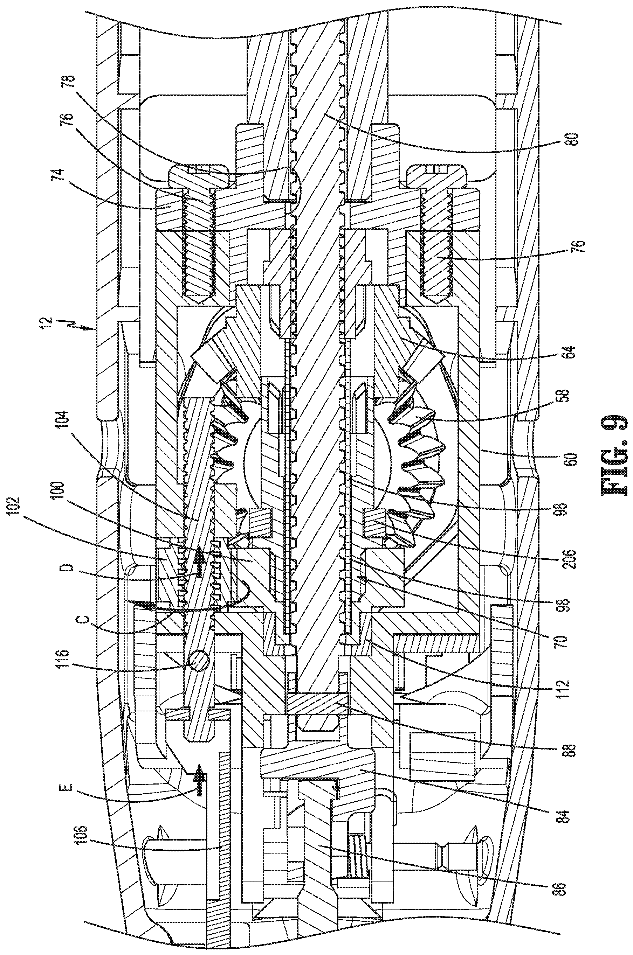

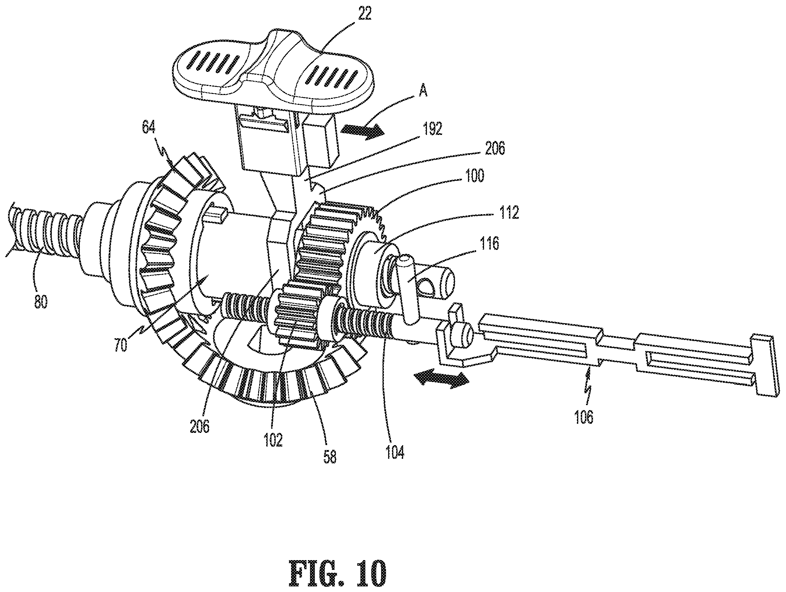

FIGS. 8-10 illustrate the stapling device 10 as the clutch switch 22 is moved to the articulation position. When the clutch switch 22 is slid in a distal direction as indicated by arrow "A" in FIG. 8, the clutch 70 is moved about the tubular spacer 98 in the direction of arrow "B" to move the splines 176 (FIG. 13) on the distal portion 162 of the clutch 70 into engagement with the splines 114 on the first articulation gear 100. As illustrated, the engaging ends of the splines 114 and 176 can be tapered to allow the splines to self-align. When the motor 54 is energized, the motor 54 rotates the first bevel gear 58 which is engaged with and rotates the second bevel gear 64. As described above, the second bevel gear 64 is engaged with the clutch 70 such that rotation of the second bevel gear 64 rotates the clutch 70. In the articulation position, the clutch 70 is engaged with the first articulation gear 100. Rotation of the first articulation gear 100 causes rotation of the second articulation gear 102 about the articulation screw 102 in the direction indicated by arrow "C" in FIG. 9 to move the articulation screw 104 longitudinally in the direction of arrow "D". The articulation screw 104 is coupled to the articulation link 106 such that longitudinal movement of the articulation screw 104 causes longitudinal movement of the articulation link 106 in the direction of arrow "E" in FIG. 9. As described above, longitudinal movement of the articulation link 106 causes longitudinal movement of the articulation body 130, the articulation plate 132, and the articulation rod 134 to articulate the tool assembly 16 in the direction of arrow "F" in FIG. 14.

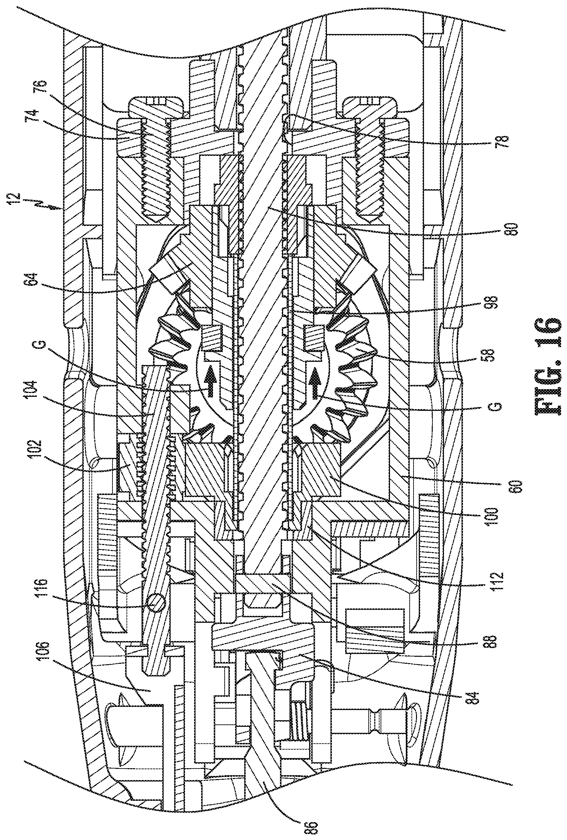

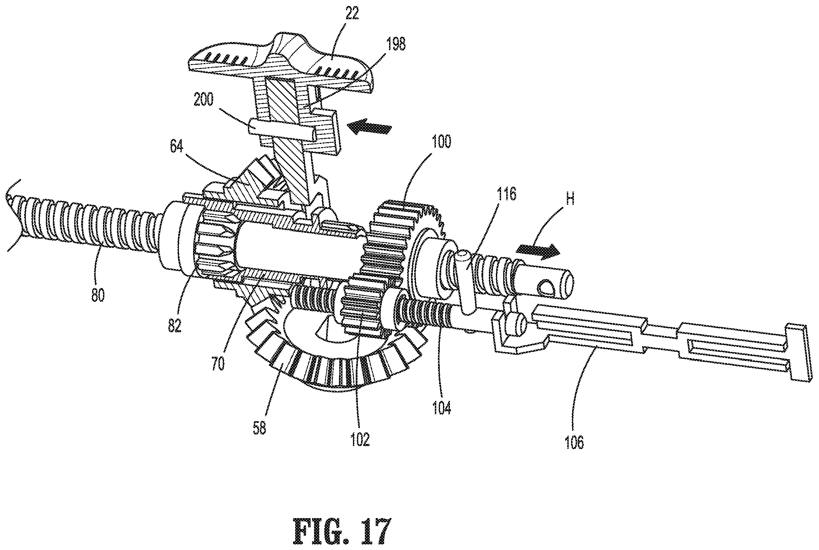

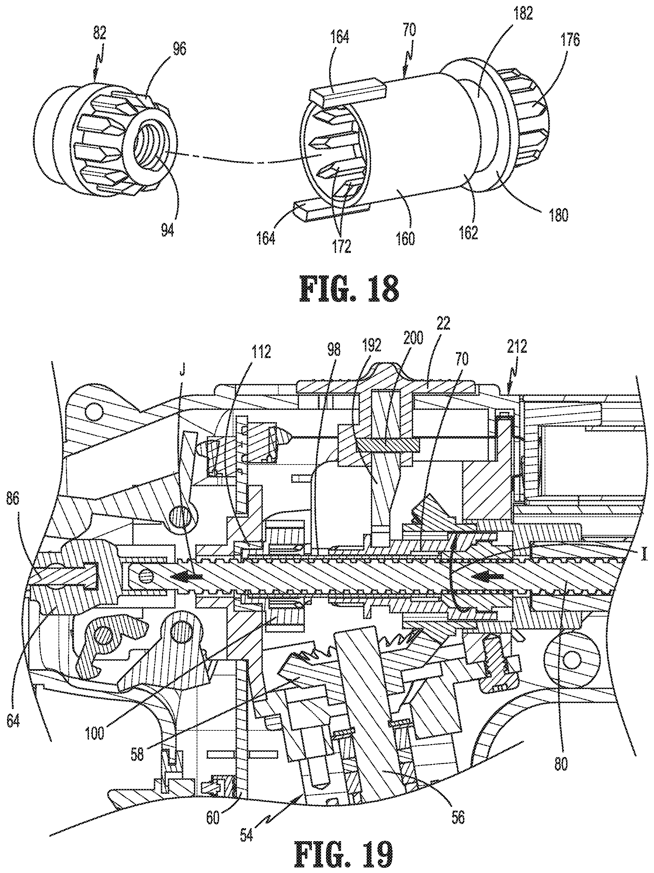

FIGS. 15-20 illustrate the clutch 70 in the clamp/fire position. When the clutch switch 22 is moved proximally along the outer surface of the housing 40 in the direction of arrow "G" in FIG. 15, the clutch 70 is moved proximally along the tubular spacer 98 in the direction of arrows "H" in FIG. 15 to move the splines 172 (FIG. 18) of the clutch 70 into engagement with the splines 96 (FIG. 18) of the screw nut 82. When this occurs, the clutch 70 will disengage from the first articulation gear 100. In the clamp/fire position, when the motor 54 is energized, the motor 54 rotates the first bevel gear 58 which is engaged with and rotates the second bevel gear 64. As described above, the second bevel gear 64 is engaged with the clutch 70 such that rotation of the second bevel gear 64 rotates the clutch 70 in the direction of arrow "I" in FIG. 19. As the clutch 70 is rotated, the screw nut 82 also rotates about the drive screw 80 to move the drive screw 80 longitudinally in the direction of arrow "J". As described above, the drive screw 80 is coupled to the drive rod 86 and moves the drive rod 86 longitudinally within the adapter assembly 14 (FIG. 1).

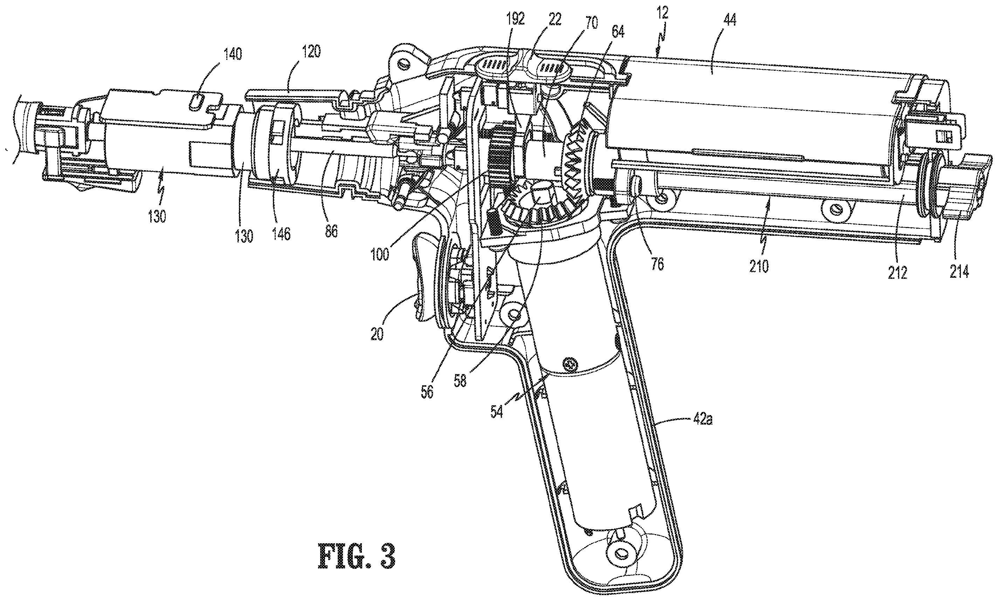

The PCB 62 (FIG. 5) is electrically coupled to battery pack 44, the motor 54, the actuation button 20, and the safety switch assembly 230. The PCB 62 accommodates a motor controller, switches that are coupled to the actuation button 20 (FIG. 3), and a processor or controller that controls operation of the handle assembly 10 to control actuation of the tool assembly 16 (FIG. 1). The controller can include any suitable electrical components for operating the disclosed surgical device or components thereof. The controller may include any type of computing device, computational circuit, or any type of processor or processing circuit capable of executing a series of instructions that are stored in memory. The controller may include multiple processors and/or multicore central processing units (CPUs) and may include any type of processor, such as a microprocessor, digital signal processor, microcontroller, programmable logic device (PLD), field programmable gate array (FPGA), or the like. The controller may also include a memory to store data and/or instructions that, when executed by the one or more processors, cause the one or more processors to perform one or more methods and/or algorithms.

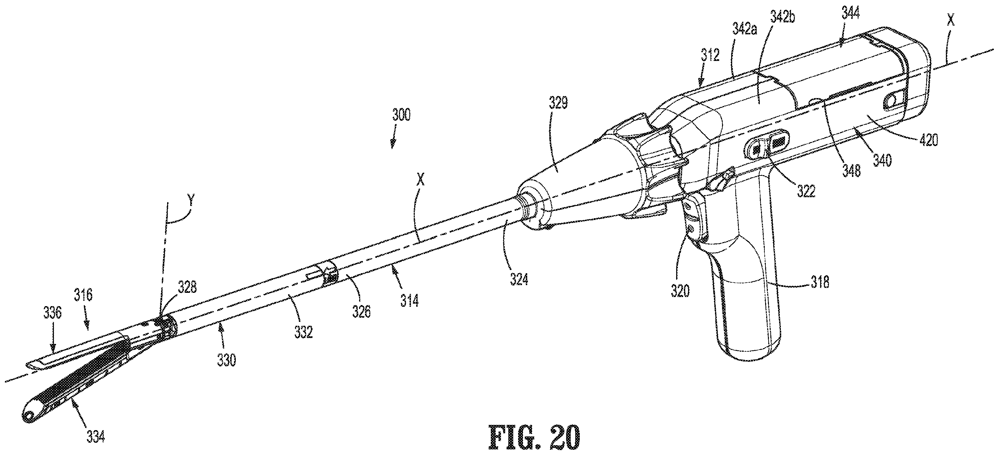

FIGS. 20-32 illustrate an alternate version of the stapling device 10 (FIG. 1) shown generally as stapling device 300. FIG. 20 illustrates the stapling device 300 which includes a handle assembly 312, an elongate body or adapter assembly 314, and a tool assembly 316. The handle assembly 312 includes a stationary handle portion 318, an actuation button 320, and clutch switches 322. One clutch switch is positioned on each side of the handle assembly 312. The adapter assembly 314 defines a longitudinal axis "X" and includes a proximal portion 324 that is coupled to the handle assembly 312, and a distal portion 326 that supports the tool assembly 316. The tool assembly 316 is secured to the distal portion 326 of the adapter assembly 314 by a pivot member 328 that defines an axis "Y" that is transverse to the longitudinal axis "X". The tool assembly 16 can articulate about the axis "Y" between an articulated position in which the tool assembly 16 is aligned with the longitudinal axis "Y" and non-articulated positions in which a longitudinal axis of the tool assembly and the longitudinal axis "X" define acute angles. The proximal portion 324 of the adapter assembly 314 is supported within a rotation knob 329 that is rotatably coupled to a distal portion of the handle assembly 312. The rotation knob 329 is manually rotatable about the longitudinal axis "X" to rotate the adapter assembly 314 and the tool assembly 316 about the longitudinal axis "X" in relation to the handle assembly 312.

In aspects of the disclosure, the tool assembly 316 forms part of a reload assembly 330 that includes a proximal body portion 332 having a proximal end and a distal end. The proximal end of the proximal body portion 332 is releasably coupled to the distal portion 326 of the adapter assembly 14 and the distal end of the proximal body portion 332 supports the tool assembly 316. The reload assembly 330 can be removed from the adapter assembly 314 and replaced to facilitate reuse of the adapter assembly 314 and handle assembly 12 during a surgical procedure. Alternately, the tool assembly 316 can be secured directly to the distal portion 326 of the adapter assembly 314.

I aspects of the disclosure, the tool assembly 316 is a stapling device and includes a cartridge assembly 334 and an anvil 336 that are movable in relation to each other between open and clamped positions. In aspects of the disclosure, the anvil 336 is fixedly secured to the proximal body portion 332 of the reload assembly 330 and the cartridge assembly 334 pivots in relation to the anvil assembly 326 and proximal body portion 332 between the open and clamped positions. It is envisioned that the cartridge assembly 334 can be fixedly mounted to the proximal body portion 332 of the reload assembly 330 and the anvil 336 can pivot between the open and clamped positions. Although the tool assembly 316 is illustrated in the form of a stapling device, it is envisioned that the tool assembly 316 may include a variety of different types of surgical devices including graspers, vessel sealers, clip appliers, stitching devices or the like.

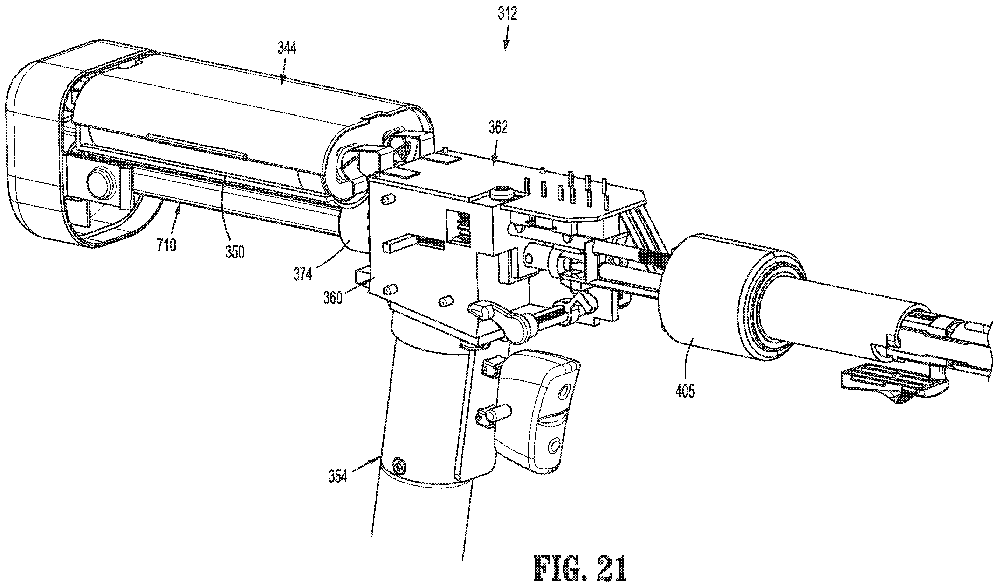

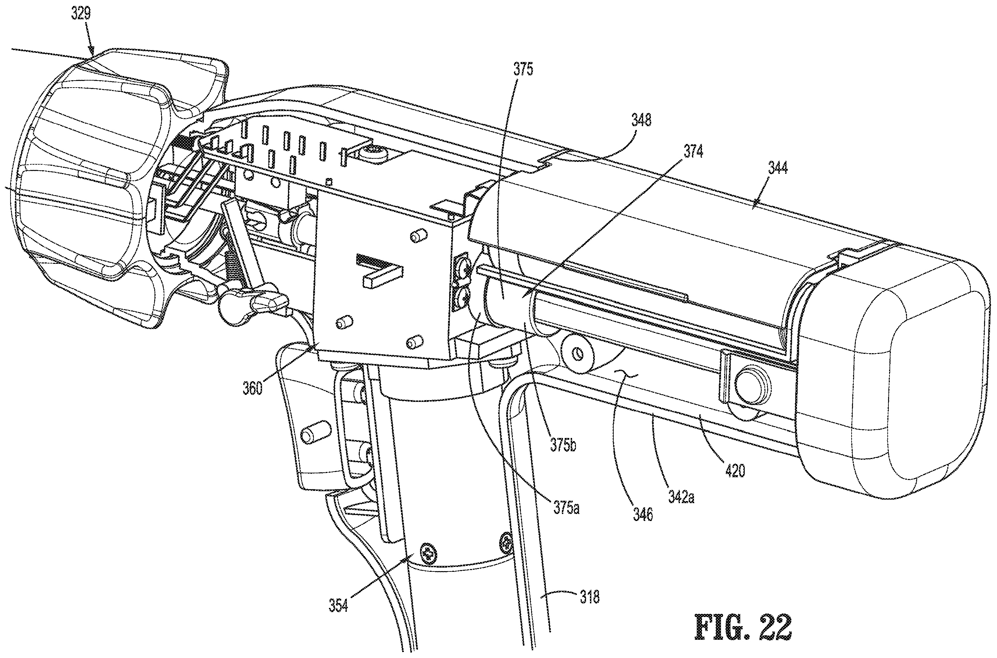

FIGS. 21-24 illustrate the handle assembly 312 of the stapling device 310 which includes a body 340 (FIG. 20) formed of first and second half-sections 342a and 342b and a battery pack 344. The first and second half-sections 342a and 342b are coupled together to form the stationary handle portion 318 (FIG. 20) and define a cavity 346 (FIG. 22) and a recess 348 (FIG. 20) that receives the battery pack 344. The battery pack 344 is substantially similar to the battery pack 44 (FIG. 5) and will not be described in further detail herein.

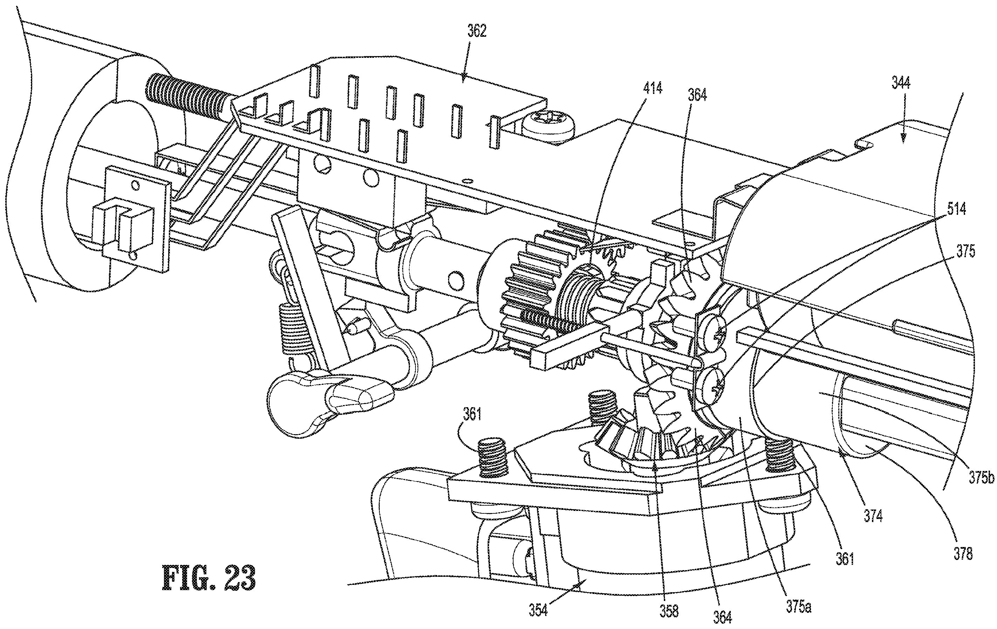

The stationary handle portion 318 of the body 340 of the handle assembly 212 defines a portion of the cavity 346 (FIG. 22) and receives a motor 354 (FIG. 21) that has a motor shaft (not shown). The motor shaft is secured to a first bevel gear 358 (FIG. 23) such that operation of the motor 354 causes rotation of the first bevel gear 358. The handle assembly 312 includes a gear housing 360 (FIG. 21) that is secured above the stationary handle portion 318 of the body 340 of the handle assembly 312 to the body 340 with, e.g., screws 361 (FIG. 23) such that the first bevel gear 358 is positioned within the gear housing 360. The gear housing 360 has a top surface as viewed in FIG. 21 that supports a printed circuit board 362 ("PCB") which is electrically coupled to the actuation button 320 and to the battery pack 344.

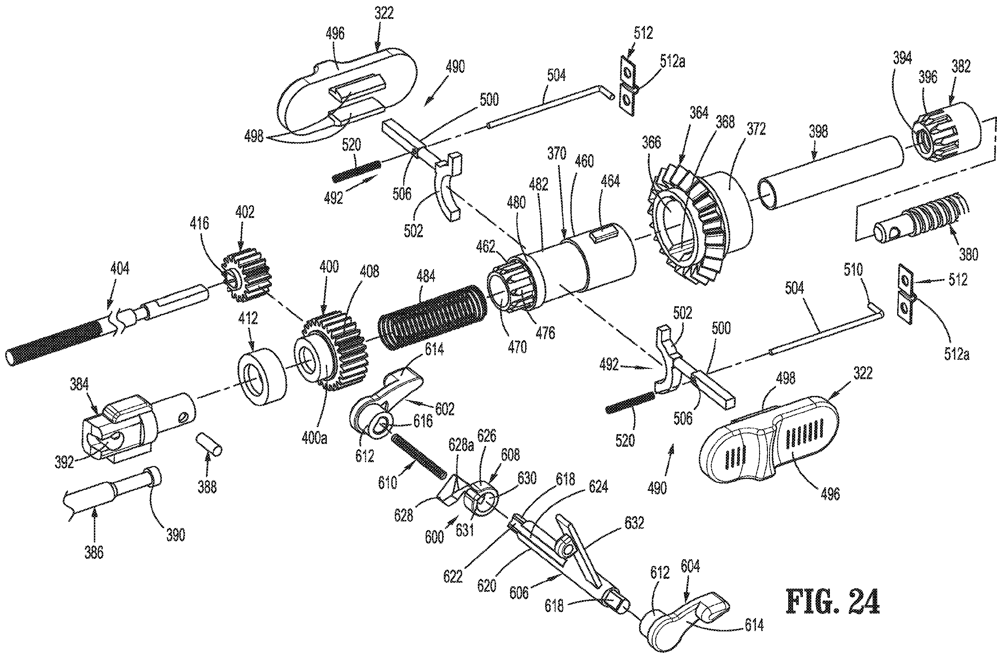

The handle assembly 312 includes a second bevel gear 364 (FIG. 23) that is engaged with the first bevel gear 358 and is positioned within the gear housing 360. The second bevel gear 364 defines a central through bore 366 (FIG. 24) and longitudinal slots 368 (FIG. 24) that extend along a length of the central through bore 366. The longitudinal slots 368 are provided to couple the second bevel gear 364 to a clutch 370 described in further detail below. The second bevel gear 364 is coupled to the first bevel gear 358 such that rotation of the first bevel gear 358 about a first axis causes rotation of the second bevel gear 364 about a second axis that is substantially perpendicular to the first axis.

The handle assembly 312 includes a support member 374 (FIG. 23) that is secured to the gear housing 360 and includes a stepped cylindrical body 375 that defines a through bore 378. The stepped cylindrical body 375 (FIG. 22) has a distal portion 375a and a proximal portion 375b. The distal portion 375a of the support member 374 extends through an opening in the gear housing 360 into the gear housing 360. The second bevel gear 364 (FIG. 24) includes a proximal hub portion 372 (FIG. 24) that is supported for rotation within the distal portion 375a (FIG. 23) of the support member 374. In aspects of the disclosure, the support member 374 is fixedly secured to the gear housing 360 within the body 340 of the handle assembly 312 by screws or the like.

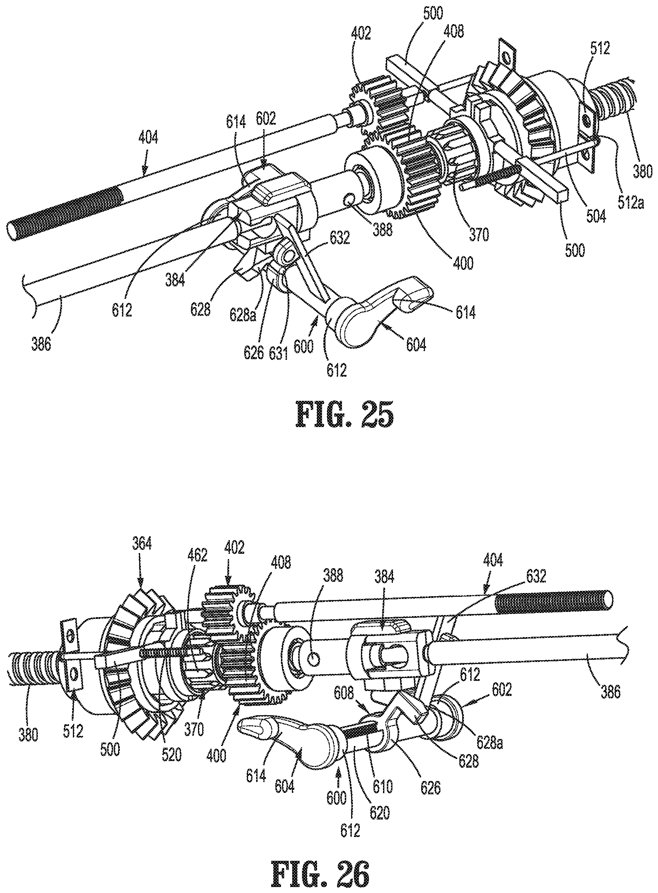

FIGS. 24-26 illustrate components of a drive assembly of the handle assembly 312 (FIG. 20). The drive assembly includes a drive screw 380, a screw nut 382 (FIG. 24), a coupling member 384, and a drive rod 386. The drive screw 380 extends through the through bore 378 of the support member 374 (FIG. 22) and has a proximal portion and a distal portion. The distal portion of the drive screw 380 is fixedly coupled to a proximal portion of the coupling member 384 by a pin 388 (FIG. 25). The coupling member 384 includes a distal portion that is coupled to a proximal portion of the drive rod 386. In aspects of the disclosure, the proximal portion of the drive rod 386 includes a stepped portion 390 that is received within a slot 392 (FIG. 6) in the distal portion of the coupling member 384 to axially fix the drive rod 386 to the coupling member 384 but allow the drive rod 386 to rotate in relation to the coupling member 384. The components on the drive assembly are coupled such that the longitudinal movement of the drive screw 380 causes longitudinal movement of the drive rod 386.

The screw nut 382 is received partly within the central through bore 366 (FIG. 24) of the second bevel gear 364 and within the distal portion 375a (FIG. 23) of the support member 364. The screw nut 382 defines a threaded through bore 394 and includes external gear teeth or splines 396 (FIG. 24). The threaded through bore 394 receives the drive screw 380 to threadedly couple the screw nut 382 about the drive screw 380. The splines 396 engage the clutch 370 when the clutch 370 is in a clamp/fire position as described in further detail below.

A tubular spacer 398 is received about the drive screw 380 and engages a distal portion of the screw nut 382 to maintain the axial position of the screw nut 382 within the body 340 (FIG. 1) of the handle assembly 312. The tubular spacer 398 also supports the clutch 370 for longitudinal movement between the clamp/fire position and an articulation position as described in further detail below.

The handle assembly 312 (FIG. 20) includes an articulation mechanism that includes a first articulation gear 400, a second articulation gear 402, an articulation screw 404, and an articulation link (not shown). The first articulation gear 400 is supported for rotation on the gear housing 360 and includes outer gear teeth 408. In aspects of the disclosure, the first articulation gear 400 includes a distally extending hub 400a (FIG. 24) that is rotatably supported on a bearing 412 (FIG. 24) supported on the gear housing 360 (FIG. 21). The first articulation gear 400 and the bearing 412 define central openings that facilitate passage of the drive screw 380 to a position distally of the first articulation gear 400 and the bearing 412. The central opening of the first articulation gear 400 is defined by an annular array of splines 414 (FIG. 23) that are engaged by the clutch 370 when the clutch 370 is moved to the articulation position.

The second articulation gear 402 is rotatably supported on the gear housing 360 and is engaged with the first articulation gear 400. The second articulation gear 402 defines a non-circular through bore 416 that receives a non-circular end of the articulation screw 404 to rotatably fix the second articulation gear 402 to the articulation screw 404. The second articulation gear 402 is axially fixed to the gear housing 360 within the body 340 of the handle assembly 312 (FIG. 1) such rotation of the second articulation gear 402 causes rotational movement of the articulation screw 404. The articulation screw 404 includes a distal portion that is coupled to a proximal portion of the articulation link (not shown) by a coupling 405 (FIG. 21) such that rotational movement of the articulation screw 404 causes longitudinal movement of the articulation link within the elongate body 314 (FIG. 20) of the stapling device 310.

Although not described in detail herein, the stapling device 300 includes an articulation assembly similar to the articulation assembly 124 shown in FIG. 5 and described in detail above which connects the articulation screw 404 to the tool assembly 316 (FIG. 20). The articulation mechanism of the handle assembly 312 (FIG. 1) operates in a manner similar to the articulation mechanism of the handle assembly 12 (FIG. 1) and will not be described in further detail herein.

The clutch 370 is slidably positioned about the tubular spacer 398 (FIG. 24) within the through bore 366 of the second bevel gear 364 and is movable between an articulation position and a clamp/fire position. In the articulation position, the clutch 370 is engaged with the first articulation gear 400 such that rotation of the clutch 370 effects rotation of the first articulation gear 400 to move the tool assembly 316 (FIG. 20) between non-articulated and articulated positions as described above regarding the stapling device 10. In the clamp/fire position, the clutch 370 is engaged with the screw nut 382 such that rotation of the clutch 370 causes rotation of the screw nut 382 moves the drive assembly including the drive rod 386 longitudinally within the stapling device 300 to actuate the tool assembly 316 (FIG. 20) as described above regarding the stapling device 10.

FIG. 24 illustrates the clutch 370 and the second bevel gear 364. The clutch 370 includes a proximal portion 460 and a distal portion 462. The proximal portion 460 of the clutch 370 includes raised extensions 464 that extend in a longitudinally towards the screw nut 382 and are received within the longitudinal slots 368 in the second bevel gear 364. Receipt of the raised extensions 464 within the longitudinal slots 368 of the second bevel gear 364 rotatably couples the second bevel gear 364 to the clutch 370. Thus, when the motor 354 (FIG. 22) is energized to rotate the first bevel gear 358 and the second bevel gear 364 as described above, the clutch 370 rotates with the second bevel gear 364.

The clutch 370 defines a through bore 470 and includes internal splines (not shown) like splines 172 (FIG. 11) of the clutch 70. When the clutch 370 is in its clamp/fire position, the splines on the inner surface of the clutch 370 are engaged with the splines 396 on the screw nut 382. As such, when the motor 54 (FIG. 22) is energized to rotate the clutch 370, the screw nut 382 is also rotated. The distal portion 462 of the clutch 370 includes an annular flange 480 and defines an annular channel 482.

The clutch 370 and the first articulation gear 400 are rotatably coupled to each other when the clutch 370 is in the articulation position. More specifically, the distal portion of the clutch 370 supports a plurality of splines 476. When the clutch 370 is in the articulation position (FIG. 8), the splines 476 on the clutch 470 are engaged with the splines 414 (FIG. 23) of the first articulation gear 400 such that rotation of the clutch 470 causes rotation of the first articulation gear 400.

The clutch 470 is biased towards the clamp/fire position by a biasing member 484. In aspects of the disclosure, the biasing member 484 includes a coil spring although other types of biasing members are envisioned. The biasing member 484 is compressed between a distal face of the clutch 370 and the first articulation gear 400 such that the clutch 370 is urged proximally towards the screw nut 382.

The handle assembly 312 includes a selector switch assembly 490 that is similar to the selector switch assembly 190 (FIG. 6) of the handle assembly 12 and provides a clinician a mechanism to move the clutch 370 from the clamp/fire position to the articulation position. The selector switch assembly 490 includes clutch switches 322 and fork members 492. The clutch switches 322 are slidably positioned on opposite sides of the body 340 of the handle assembly 312 (FIG. 20) and include a finger engagement member 496 and extensions 498 that extend from the finger engagement member 496 through longitudinal slots (not shown) in the body 340 of the handle assembly 312. The extensions 498 are resilient and are received through the elongated slots (not shown) in the body 340 of the housing 312 in a snap-fit manner to slidably secure the clutch switches 322 to the body 340 (FIG. 20) of the handle assembly 312. Each of the fork members 492 includes a transverse rod 500 and a semi-circular coupling member 502. Each one of the transverse rods 500 is secured to a respective one of the clutch switches 322. In aspects of the disclosure, each of the clutch switches 322 defines a bore (not shown) that receives an end of a respective one of the transverse rods 500 to secure the clutch switches 322 to the transverse rods 500. Alternately, it is envisioned that the transverse rods 500 can be secured to the clutch switches 322 in a variety of different manners or the transverse rods 500 and the clutch switches 322 can be integrally formed. Each of the semi-circular coupling members 502 of the fork members 492 is received within the annular channel 482 of the clutch 370. When the clutch switch 322 is moved longitudinally along the body 340 of the handle assembly 312, the clutch 370 is moved longitudinally within the body 340 along the tubular spacer 398 against the urging of the biasing member 484 from the clamp/fire position to the articulation position.

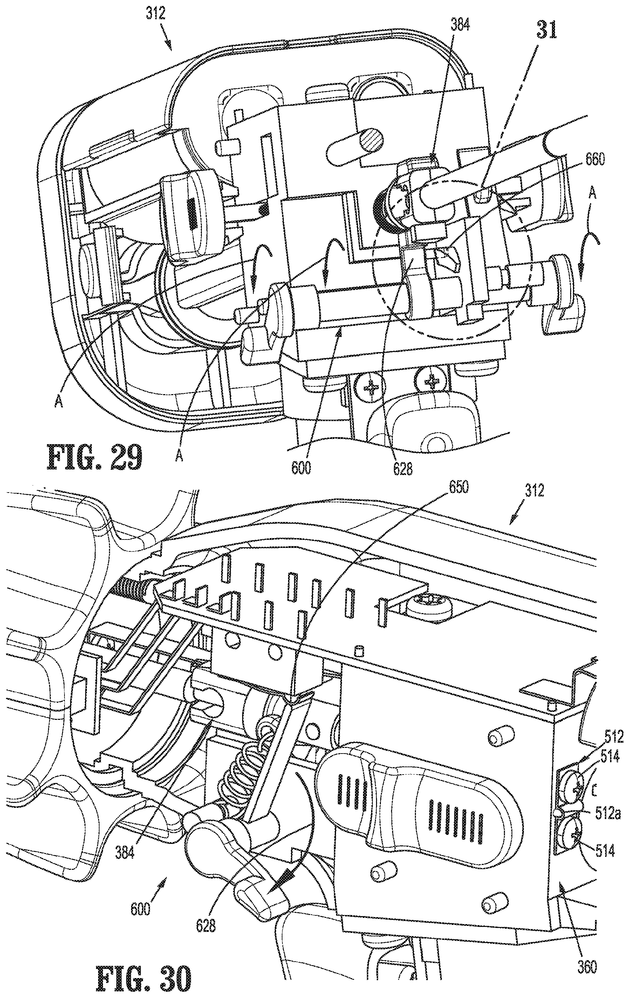

In aspects of the disclosure, the handle assembly 312 includes guide rods 504 that are supported within the body 340 of the handle assembly 312 and extend in a direction that is parallel to the longitudinal axis "X" (FIG. 20) of the elongate body 314. Each of the guide rods 504 is supported within the body 340 of the handle assembly 312 and extends through an opening 506 in a respective one of the transverse rods 500 of the selector switch assembly 490. The guide rods 504 guide movement of the fork members 492 of the selector switch assembly 490 as the selector switch assembly 490 is moved from a retracted position to an advanced position to move the clutch 370 from the clamp/fire position to the articulation position. In aspects of the disclosure, each of the guide rods 504 has an L-shaped configuration and includes a proximal transverse portion 510. The proximal transverse portion 510 is received within a slot 512a of a bracket 512 and the bracket 512 is secured to the gear housing 360 with screws 514 (FIG. 30).

In some aspects of the disclosure, the selector switch assembly 490 includes biasing members 520 that are positioned to urge the fork members 492 of the selector switch assembly 490 proximally to urge the clutch 370 towards the clamp/fire position. The biasing members 520 can be coil springs although other types of biasing members are envisioned.

FIGS. 24-26 illustrate a safety toggle mechanism 600 which includes first and second toggle members 602 and 604, a shaft 606, a safety slider 608, and a biasing member 610 (FIG. 24). Each of the safety toggles 602 and 604 includes a hub 612 and a lever 614. The hubs 612 are rotatably supported in openings (not shown) defined in opposite sides of the body 340 of the handle assembly 312 (FIG. 20) and define D-shaped bores 616 (FIG. 24). The shaft 606 has first and second ends that include D-shaped extensions 618 (FIG. 24) that are received in the D-shaped bores 616 of the respective first and second toggle members 602 and 604 to rotatably fix the first and second ends of the shaft 606 to the first and second toggle members 602 and 604. The shaft 606 includes a reduced diameter portion 620 that defines a channel 622 (FIG. 24) and an elongated slot 624 that communicates with the channel 622. The safety slider 608 has a circular body 626 and a stop member 628 that extends outwardly from the circular body 626 and includes a tapered cam surface 628a. The circular body 626 of the safety slider 608 is slidably positioned about the reduced diameter portion 620 of the shaft 606 and includes an inwardly extending tab 630 and a protrusion 631 that extends towards the second toggle member 604. The tab 630 extends through the elongated slot 624 into the channel 622 of the shaft 606 to prevent the safety slider 608 from rotating about the shaft 606. The biasing member 610 is positioned between the first toggle member 602 and the tab 630 of the safety slider 608 within the channel 622 of the shaft 606 to urge the safety slider 608 about the reduced diameter portion 620 of the shaft 606 towards the second toggle 604.

The shaft 606 of the safety toggle mechanism 600 supports an arm 632 that extends outwardly from the shaft 606 within the body 340 (FIG. 20) of the handle assembly 312. The shaft 606 is rotatable within the cavity 346 (FIG. 22) of the body 340 (FIG. 1) by rotating the first and second toggle members 602 and 604 from either side of the handle assembly 312 to move the safety toggle mechanism 600 from an inactive position to an active position (FIG. 30). When the safety toggle mechanism 600 is in the active position, the arm 632 is rotated into engagement with a contact 650 (FIG. 30) within the handle assembly 312 to close the contact 650 and place the stapling device 10 (FIG. 1) in a fire-ready position as described in further detail below.

As shown in FIGS. 25 and 26, when the drive assembly of the stapling device 300 (FIG. 1) is in a retracted position, the coupling member 384 is positioned adjacent the stop member 628 of the safety slider 608 to prevent rotation of the safety toggle mechanism 600 from the inactive position to the active position. As such, the stop member 628 prevents the arm 632 on the shaft 606 from moving into engagement with the contact 650 to close the contact 650. Thus, with the drive assembly of the stapling device 10 (FIG. 1) in the retracted position, the stapling device 300 cannot be fired.

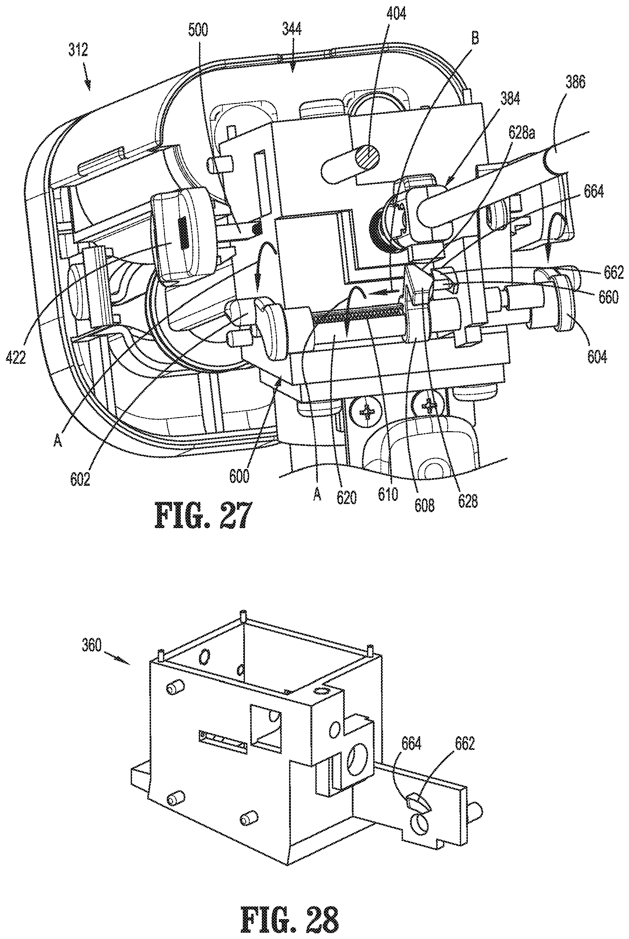

FIGS. 27-31 illustrate the handle assembly 312 as the safety toggle mechanism 600 is moved from the inactive position to the active position. When the drive assembly is moved towards its advanced position to advance the coupling member 384 distally past the stop member 628 of the safety slider 608 and move the tool assembly 316 to the clamped position, the first and second toggle members 602 and 604 can be rotated in the direction of arrows "A" in FIGS 27 and 29 and 30. When the safety toggle members 602 and 604 are rotated in the direction of arrows "A", the stop member 628 of the safety slider 608 rotates past the coupling member 384 and the arm 632 supported on the shaft 606 moves into engagement with the contact 650 (FIG. 30) to move the contact 650 to a closed position and place the stapling device 300 in a fire-ready position. With the contact 650 closed, the stapling device 300 (FIG. 20) can be actuated by depressing the actuation buttons 320.

As the abutment member 628 of the safety slider 608 moves in the direction of arrow "A", a projection 660 formed on the safety slider 608 engages and moves along a tapered cam member 662 (FIG. 28) formed on the gear housing 360. This engagement cams the safety slider 608 in the direction of arrow "B" in FIG. 27 against the urging of the biasing member 610 along the reduced diameter portion of the shaft 606 past the tapered cam member 662. When the projection 660 moves past the tapered cam member 662, the biasing member 610 returns the safety slider 608 to its original position on the reduced diameter portion 620 of the shaft 606. The tapered cam member 662 has a proximal stop surface 664 (FIG. 28). When the projection 660 passes off of the tapered cam member 662, the projection 660 engages the stop surface 664 on the gear housing 360 to retain the safety toggle mechanism 600 in the active position. As stated above, in the active position, the contact 650 is in a closed position to place the stapling device 300 in a fire-ready position.

The stapling device 300 includes a manual retraction mechanism 710 (FIG. 21) which is substantially like the manual retraction mechanism 210 (FIG. 5) of the handle assembly 12 (FIG. 1) of the surgical stapling device 10 (FIG. 1).

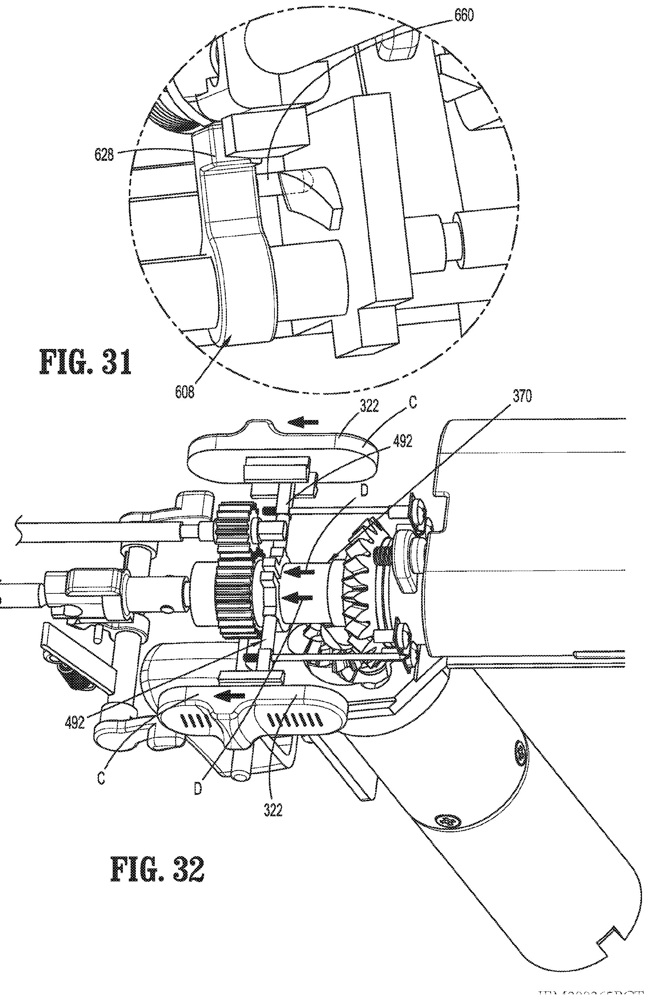

FIG. 32 illustrates the operation of the selector switch assembly 490. As detailed above, the clutch 370 is urged by the biasing member 484 (FIG. 24) (and to a lesser extent by biasing members 520) towards the clamp/fire position. In order to move the clutch 370 to the articulation position, one or both of the clutch switches 322 can be engaged by a clinician and slid distally along the body 340 (FIG. 20) of the handle assembly 312 in the direction of arrows "C" to move the fork members 492 in the direction of arrows "C". The fork members 492 are secured about the clutch 370 such that movement of the fork members 492 in the direction of arrows "C" moves the clutch 370 in the direction of arrows "D" to the articulation position in which the splines 476 (FIG. 24) on the distal portion of the clutch 370 are engaged with the splines 414 (FIG. 23) of the first articulation gear 400. When the selector switch assembly 490 is released by a clinician, the biasing member 484 will urge the clutch 370 back to the clamp/fire position. Operation of the stapling device 300 (FIG. 20) with the clutch 370 in the clamp/fire position and the articulation position is substantially as described above and will not be described in further detail herein.

Persons skilled in the art will understand that the devices and methods specifically described herein and illustrated in the accompanying drawings are non-limiting exemplary aspects of the disclosure. It is envisioned that the elements and features illustrated or described in connection with one exemplary embodiment may be combined with the elements and features of another without departing from the scope of the present disclosure. As well, one skilled in the art will appreciate further features and advantages of the disclosure based on the above-described aspects of the disclosure. Accordingly, the disclosure is not to be limited by what has been particularly shown and described, except as indicated by the appended claims.

## Claims

1. A handle assembly (312) for a surgical device comprising:
a body (340) supporting at least one clutch switch (322);
a drive assembly supported within the housing, the drive assembly including a drive screw (380), a screw nut (382), and a drive rod (386), the drive rod having a proximal portion and a distal portion, the screw nut defining a threaded bore (394) and including external splines (396), the drive screw extending through the threaded bore, and the drive rod having a proximal portion coupled to the drive screw;
an articulation mechanism supported within the housing, the articulation mechanism including a first articulation gear (400) , a second articulation gear (402) and an articulation screw (404), the first articulation gear defining a through bore and including splines positioned within the through bore and outer gear teeth (408), the second articulation gear including outer gear teeth that are engaged with the outer gear teeth of the first articulation gear;
a clutch (470) supported within the housing between the first articulation gear and the screw nut, the clutch movable between a clamp/fire position in which the clutch is engaged with the external splines of the screw nut and an articulation position in which the clutch is engaged with the splines of the first articulation gear;
a biasing member (484) supported within the housing, the biasing member urging the clutch towards the clamp/fire position; and
a motor (354) coupled to the clutch, the motor operable to rotate the clutch within the housing to actuate one of the drive assembly or the articulation mechanism.

2. The handle assembly of claim 1, further including a first bevel gear (358) coupled to the motor and a second bevel gear (364) coupled to the first bevel gear.

3. The handle assembly of claim 2, wherein the second bevel gear defines a through bore (366) and a longitudinal slot that communicates with the through bore, the through bore of the second bevel gear receiving the screw nut and the drive screw, and the clutch including a raised extension (464) that is received within the longitudinal slot to rotatably couple the clutch to the second bevel gear.

4. The handle assembly of claim 1, wherein the articulation mechanism includes an articulation link that is coupled to the articulation screw such that movement of the articulation screw causes longitudinal movement of the articulation link.

5. The handle assembly of claim 1 wherein the at least one clutch switch (322) includes a clutch switch on each side of the body, the clutch switches coupled to the clutch by forked members, and the clutch switches movable along the body to move the clutch from the clamp/fire position to the articulation position.

6. The handle assembly of claim 5, wherein the body supports guide rods (504) and the forked members define openings, the guide rods extending through the openings in the forked members to guide movement of the forked members as the clutch is moved from the clamp/fire position to the articulation position.

7. The handle assembly of claim 1, further including a safety toggle mechanism (600) supported on the body, the safety toggle mechanism including at least one toggle member (602, 604) and a shaft (606) coupled to the at least one toggle member, the shaft being rotatable in response to manipulation of the at least one toggle member to move the safety toggle mechanism from an inactive position in which the handle assembly is deactivated to an active position in which the handle assembly is activated; and wherein body of the handle assembly supports a contact (650) and the shaft of the safety toggle mechanism includes an arm, wherein in the inactive position of the safety toggle mechanism, the arm is spaced from the contact and the contact is in an open position, and in the active position of the safety toggle mechanism, the arm is engaged with the contact and the contact is in a closed position.

8. The handle assembly of claim 7, wherein the safety toggle mechanism includes a slider (608) mounted on the shaft, the slider including a stop member (628); and wherein the drive assembly includes a coupling member (384) that couples the drive screw to the drive rod, the drive screw, the coupling member and the drive rod movable between retracted and advanced positions within the body in response to actuation of the motor when the clutch is in the clamp/fire position.

9. The handle assembly of claim 8, wherein the coupling member is positioned to obstruct movement of the safety toggle mechanism from the inactive position to the active position when the coupling member is in its retracted position; and wherein body supports a tapered cam member (622) having a tapered cam surface (628a) and proximal stop surface (684) , and the slider includes a projection (660) , the projection movable along the tapered cam surface to move the slider from a first position on the shaft in which the slider is aligned with the tapered cam member to a second position on the shaft in which the slider is positioned outwardly of the tapered cam member as the safety toggle mechanism is moved from the inactive position to the active position, wherein the projection is aligned with the proximal stop surface when the safety toggle mechanism is in the active position to retain the safety toggle mechanism in the active position.

10. The handle assembly of claim 9, wherein the slider is urged towards the first position by a biasing mechanism.

11. A surgical device comprising:
a handle assembly as claimed in claim 1 and
an adapter assembly (314) defining a longitudinal axis and having a proximal portion (324) and a distal portion (326) , the adapter assembly including an articulation rod (134) , the proximal portion of the adapter assembly coupled to the handle assembly, the articulation rod having a proximal portion coupled to the articulation screw and a distal portion, the drive rod extending through the adapter assembly; and
a tool assembly (316) pivotably coupled to the distal portion of the adapter assembly about an axis that is transverse to the longitudinal axis of the adapter assembly, the distal portion of the articulation rod coupled to the tool assembly to pivot the tool assembly between a non-articulated position in which the tool assembly is aligned with the longitudinal axis and articulated positions in which the tool assembly is misaligned with the longitudinal axis.

12. The surgical device of claim 11, wherein the at least one clutch switch includes a clutch switch supported on each side of the body of the handle assembly, the clutch switches coupled to the clutch and movable along the body of the handle assembly to move the clutch from the clamp/fire position to the articulation position; and further including a safety toggle mechanism supported on the body, the safety toggle mechanism including at least one toggle member and a shaft coupled to the at least one toggle member, the shaft rotatable in response to manipulation of the at least one toggle member to move the safety toggle mechanism from an inactive position in which the handle assembly is deactivated to an active position in which the handle assembly is activated.

13. The surgical device of claim 12, wherein body of the handle assembly supports a contact and the shaft of the safety toggle mechanism includes an arm, wherein in the inactive position of the safety toggle mechanism, the arm is spaced from the contact and the contact is in an open position, and in the active position of the safety toggle mechanism, the arm is engaged with the contact and the contact is in a closed position; and wherein the safety toggle mechanism includes a slider mounted on the shaft, the slider including a stop member.

14. The surgical device of claim 13 wherein the drive assembly includes a coupling member that couples the drive screw to the drive rod, the drive screw, the coupling member and the drive rod movable between retracted and advanced positions within the body in response to actuation of the motor when the clutch is in the clamp/fire position; and wherein the coupling member is positioned to obstruct movement of the safety toggle mechanism from the inactive position to the active position when the coupling member is in its retracted position.

## Patentansprüche

1. Griffanordnung (312) für eine chirurgische Vorrichtung, die Folgendes umfasst:
einen Körper (340), der mindestens einen Kupplungsschalter (322) trägt;
eine Antriebsanordnung, die innerhalb des Gehäuses getragen wird, wobei die Antriebsanordnung eine Antriebsschraube (380), eine Schraubenmutter (382) und eine Antriebsstange (386) beinhaltet, wobei die Antriebsstange einen proximalen Abschnitt und einen distalen Abschnitt aufweist, wobei die Schraubenmutter eine Gewindebohrung (394) definiert und Außenverzahnungen (396) beinhaltet, wobei sich die Antriebsschraube durch die Gewindebohrung erstreckt und die Antriebsstange einen proximalen Abschnitt aufweist, der mit der Antriebsschraube gekoppelt ist;
einen Gelenkmechanismus, der innerhalb des Gehäuses gelagert ist, wobei der Gelenkmechanismus ein erstes Gelenkzahnrad (400), ein zweites Gelenkzahnrad (402) und eine Gelenkschraube (404) beinhaltet, wobei das erste Gelenkzahnrad eine Durchgangsbohrung definiert und Keilnuten beinhaltet, die innerhalb der Durchgangsbohrung und der äußeren Zahnradzähne (408) positioniert sind,
wobei das zweite Gelenkzahnrad äußere Zahnradzähne aufweist, die mit den äußeren Zahnradzähnen des ersten Gelenkzahnrads in Eingriff stehen;
eine Kupplung (470), die innerhalb des Gehäuses zwischen dem ersten Gelenkzahnrad und der Schraubenmutter gelagert ist, wobei die Kupplung zwischen einer Klemm-/Auslöseposition, in der die Kupplung mit den Außenverzahnungen der Schraubenmutter in Eingriff steht, und einer Gelenkposition, in der die Kupplung mit den Verzahnungen des ersten Gelenkzahnrads in Eingriff steht, bewegbar ist;
ein Vorspannelement (484), das innerhalb des Gehäuses getragen wird, wobei das Vorspannelement die Kupplung in Richtung der Klemm-/Auslöseposition drückt; und
einen Motor (354), der mit der Kupplung gekoppelt ist, wobei der Motor betreibbar ist, um die Kupplung innerhalb des Gehäuses zu drehen, um entweder die Antriebsanordnung oder den Gelenkmechanismus zu betätigen.

2. Griffanordnung nach Anspruch 1, ferner umfassend ein erstes Kegelrad (358), das mit dem Motor gekoppelt ist, und ein zweites Kegelrad (364), das mit dem ersten Kegelrad gekoppelt ist.

3. Griffanordnung nach Anspruch 2, wobei das zweite Kegelrad eine Durchgangsbohrung (366) und einen Längsschlitz definiert, der mit der Durchgangsbohrung in Verbindung steht, wobei die Durchgangsbohrung des zweiten Kegelrads die Schraubenmutter und die Antriebsschraube aufnimmt und die Kupplung eine erhöhte Verlängerung (464) beinhaltet, die innerhalb des Längsschlitzes aufgenommen ist, um die Kupplung drehbar mit dem zweiten Kegelrad zu koppeln.

4. Griffanordnung nach Anspruch 1, wobei der Gelenkmechanismus eine Gelenkverbindung beinhaltet, die mit der Gelenkschraube gekoppelt ist, so dass eine Bewegung der Gelenkschraube eine Längsbewegung der Gelenkverbindung bewirkt.

5. Griffanordnung nach Anspruch 1, wobei der mindestens eine Kupplungsschalter (322) einen Kupplungsschalter auf jeder Seite des Körpers beinhaltet, wobei die Kupplungsschalter durch gabelförmige Elemente mit der Kupplung gekoppelt sind und die Kupplungsschalter entlang des Körpers bewegbar sind, um die Kupplung von der Klemm-/Auslöseposition in die Gelenkposition zu bewegen.

6. Griffanordnung nach Anspruch 5, wobei der Körper Führungsstangen (504) trägt und die gabelförmigen Elemente Öffnungen definieren, wobei sich die Führungsstangen durch die Öffnungen in den gabelförmigen Elementen erstrecken, um die Bewegung der gabelförmigen Elemente zu führen, wenn die Kupplung von der Klemm-/Auslöseposition in die Gelenkposition bewegt wird.

7. Griffanordnung nach Anspruch 1, die ferner einen Sicherheitsumschaltmechanismus (600) beinhaltet, der an dem Körper getragen wird, wobei der Sicherheitsumschaltmechanismus mindestens ein Umschaltelement (602, 604) und eine Welle (606) beinhaltet, die mit dem mindestens einen Umschaltelement gekoppelt ist, wobei die Welle als Reaktion auf eine Manipulation des mindestens einen Umschaltelements drehbar ist, um den Sicherheitsumschaltmechanismus aus einer inaktiven Position, in der die Griffanordnung deaktiviert ist, in eine aktive Position, in der die Griffanordnung aktiviert ist, zu bewegen; und wobei der Körper der Griffanordnung einen Kontakt (650) trägt und die Welle des Sicherheitsumschaltmechanismus einen Arm beinhaltet, wobei in der inaktiven Position des Sicherheitsumschaltmechanismus der Arm von dem Kontakt beabstandet ist und sich der Kontakt in einer offenen Position befindet und sich in der aktiven Position des Sicherheitsumschaltmechanismus der Arm mit dem Kontakt in Eingriff befindet und sich der Kontakt in einer geschlossenen Position befindet.

8. Griffanordnung nach Anspruch 7, wobei der Sicherheitsumschaltmechanismus einen Schieber (608) beinhaltet, der auf der Welle montiert ist, wobei der Schieber ein Anschlagelement (628) beinhaltet; und wobei die Antriebsanordnung ein Kopplungselement (384) beinhaltet, das die Antriebsschraube mit der Antriebsstange, der Antriebsschraube, dem Kopplungselement und der Antriebsstange koppelt, die zwischen einer zurückgezogenen und einer vorgeschobenen Position innerhalb des Körpers als Reaktion auf eine Betätigung des Motors bewegbar ist, wenn sich die Kupplung in der Klemm-/Auslöseposition befindet.

9. Griffanordnung nach Anspruch 8, wobei das Kopplungselement so positioniert ist, dass es eine Bewegung des Sicherheitsumschaltmechanismus von der inaktiven Position in die aktive Position blockiert, wenn sich das Kopplungselement in seiner zurückgezogenen Position befindet; und wobei der Körper ein sich verjüngendes Nockenelement (622) mit einer sich verjüngenden Nockenfläche (628a) und einer proximalen Anschlagfläche (684) stützt, und der Schieber einen Vorsprung (660) beinhaltet, wobei der Vorsprung entlang der sich verjüngenden Nockenfläche bewegbar ist, um den Schieber von einer ersten Position auf der Welle, in der der Schieber mit dem sich verjüngenden Nockenelement ausgerichtet ist, in eine zweite Position auf der Welle zu bewegen, in der der Schieber außerhalb des sich verjüngenden Nockenelements positioniert ist, wenn der Sicherheitsumschaltmechanismus von der inaktiven Position in die aktive Position bewegt wird, wobei der Vorsprung mit der proximalen Anschlagfläche ausgerichtet ist, wenn sich der Sicherheitsumschaltmechanismus in der aktiven Position befindet, um den Sicherheitsumschaltmechanismus in der aktiven Position zu halten.

10. Griffanordnung nach Anspruch 9, wobei der Schieber durch einen Vorspannmechanismus in Richtung der ersten Position gedrückt wird.

11. Chirurgische Vorrichtung, umfassend:
eine Griffanordnung nach Anspruch 1 und
eine Adapteranordnung (314), die eine Längsachse definiert und einen proximalen Abschnitt (324) und einen distalen Abschnitt (326) aufweist, wobei die Adapteranordnung eine Gelenkstange (134) aufweist, wobei der proximale Abschnitt der Adapteranordnung mit der Griffanordnung gekoppelt ist, wobei die Gelenkstange einen proximalen Abschnitt, der mit der Gelenkschraube gekoppelt ist, und einen distalen Abschnitt aufweist, wobei sich die Antriebsstange durch die Adapteranordnung erstreckt; und
eine Werkzeuganordnung (316), die schwenkbar mit dem distalen Abschnitt der Adapteranordnung um eine Achse gekoppelt ist, die quer zu der Längsachse der Adapteranordnung ist, wobei der distale Abschnitt der Gelenkstange mit der Werkzeuganordnung gekoppelt ist, um die Werkzeuganordnung zwischen einer nicht angelenkten Position, in der die Werkzeuganordnung mit der Längsachse ausgerichtet ist, und angelenkten Positionen, in denen die Werkzeuganordnung nicht mit der Längsachse ausgerichtet ist, zu schwenken.

12. Chirurgische Vorrichtung nach Anspruch 11, wobei der mindestens eine Kupplungsschalter einen Kupplungsschalter beinhaltet, der auf jeder Seite des Körpers der Griffanordnung getragen wird, wobei die Kupplungsschalter mit der Kupplung gekoppelt sind und entlang des Körpers der Griffanordnung bewegbar sind, um die Kupplung von der Klemm-/Auslöseposition in die Gelenkposition zu bewegen; und ferner umfassend einen Sicherheitsumschaltmechanismus, der an dem Körper getragen wird, wobei der Sicherheitsumschaltmechanismus mindestens ein Umschaltelement und eine Welle umfasst, die mit dem mindestens einen Umschaltelement gekoppelt ist, wobei die Welle als Reaktion auf eine Manipulation des mindestens einen Umschaltelements drehbar ist, um den Sicherheitsumschaltmechanismus aus einer inaktiven Position, in der die Griffanordnung deaktiviert ist, in eine aktive Position, in der die Griffanordnung aktiviert ist, zu bewegen.

13. Chirurgische Vorrichtung nach Anspruch 12, wobei der Körper der Griffanordnung einen Kontakt trägt und die Welle des Sicherheitsumschaltmechanismus einen Arm beinhaltet, wobei in der inaktiven Position des Sicherheitsumschaltmechanismus der Arm von dem Kontakt beabstandet ist und sich der Kontakt in einer offenen Position befindet und sich in der aktiven Position des Sicherheitsumschaltmechanismus der Arm mit dem Kontakt in Eingriff befindet und sich der Kontakt in einer geschlossenen Position befindet; und wobei der Sicherheitsumschaltmechanismus einen Schieber beinhaltet, der auf der Welle montiert ist, wobei der Schieber ein Anschlagelement beinhaltet.

14. Chirurgische Vorrichtung nach Anspruch 13, wobei die Antriebsanordnung ein Kopplungselement beinhaltet, das die Antriebsschraube mit der Antriebsstange koppelt, wobei die Antriebsschraube, das Kopplungselement und die Antriebsstange zwischen zurückgezogenen und vorgeschobenen Positionen innerhalb des Körpers als Reaktion auf eine Betätigung des Motors bewegbar sind, wenn sich die Kupplung in der Klemm-/Auslöseposition befindet; und wobei das Kopplungselement positioniert ist, um eine Bewegung des Sicherheitsumschaltmechanismus von der inaktiven Position in die aktive Position zu blockieren, wenn sich das Kopplungselement in seiner zurückgezogenen Position befindet.

## Revendications

1. Ensemble poignée (312) pour un dispositif chirurgical comprenant :
un corps (340) supportant au moins un sélecteur d'embrayage (322) ;
un ensemble d'entraînement supporté à l'intérieur du boîtier, l'ensemble d'entraînement comprenant une vis d'entraînement (380), un écrou (382) et une tige d'entraînement (386), la tige d'entraînement possédant une partie proximale et une partie distale, l'écrou définissant un alésage fileté (394) et comportant des cannelures externes (396), la vis d'entraînement s'étendant à travers l'alésage fileté, et la tige d'entraînement possédant une partie proximale couplée à la vis d'entraînement ;
un mécanisme d'articulation supporté à l'intérieur du boîtier, le mécanisme d'articulation comportant un premier engrenage d'articulation (400), un second engrenage d'articulation (402) et une vis d'articulation (404), le premier engrenage d'articulation définissant un alésage traversant et comportant des cannelures positionnées à l'intérieur de l'alésage traversant et des dents d'engrenage extérieures (408),
le second engrenage d'articulation comportant des dents d'engrenage extérieures qui sont en prise avec les dents d'engrenage extérieures du premier engrenage d'articulation ;
un embrayage (470) supporté à l'intérieur du boîtier entre le premier engrenage d'articulation et l'écrou, l'embrayage étant mobile entre une position de serrage/déclenchement dans laquelle l'embrayage est en prise avec les cannelures externes de l'écrou et une position d'articulation dans laquelle l'embrayage est en prise avec les cannelures du premier engrenage d'articulation ;
un élément de sollicitation (484) supporté à l'intérieur du boîtier, l'élément de sollicitation poussant l'embrayage vers la position de serrage/déclenchement ; et
un moteur (354) couplé à l'embrayage, le moteur étant apte à faire tourner l'embrayage à l'intérieur du boîtier afin d'actionner l'un de l'ensemble d'entraînement ou du mécanisme d'articulation.

2. Ensemble poignée selon la revendication 1, comportant en outre un premier engrenage conique (358) couplé au moteur et un second engrenage conique (364) couplé au premier engrenage conique.

3. Ensemble poignée selon la revendication 2, le second engrenage conique définissant un alésage traversant (366) et une fente longitudinale qui communique avec l'alésage traversant, l'alésage traversant du second engrenage conique recevant l'écrou et la vis d'entraînement, et l'embrayage comportant un extension surélevée (464) qui est reçue dans la fente longitudinale pour coupler en rotation l'embrayage au second engrenage conique.

4. Ensemble poignée selon la revendication 1, le mécanisme d'articulation comportant une liaison d'articulation qui est couplée à la vis d'articulation de telle sorte que le mouvement de la vis d'articulation provoque un mouvement longitudinal de la liaison d'articulation.

5. Ensemble poignée selon la revendication 1, l'au moins un sélecteur d'embrayage (322) comportant un sélecteur d'embrayage de chaque côté du corps, les sélecteurs d'embrayage étant couplés à l'embrayage par des éléments de fourche, et les sélecteurs d'embrayage étant mobiles le long du corps pour déplacer l'embrayage de la position de serrage/déclenchement à la position d'articulation.

6. Ensemble poignée selon la revendication 5, le corps supportant des tiges de guidage (504) et les éléments de fourche définissant des ouvertures, les tiges de guidage s'étendant à travers les ouvertures dans les éléments de fourche pour guider le mouvement des éléments de fourche lorsque l'embrayage est déplacé de la position de serrage/déclenchement à la position d'articulation.

7. Ensemble poignée selon la revendication 1, comportant en outre un mécanisme de sécurité à bascule (600) supporté sur le corps, le mécanisme de sécurité à bascule comportant au moins un élément de bascule (602, 604) et un arbre (606) couplé à l'au moins un élément de bascule, l'arbre pouvant tourner en réponse à la manipulation de l'au moins un élément de bascule pour déplacer le mécanisme de sécurité à bascule d'une position inactive dans laquelle l'ensemble poignée est désactivé à une position active dans laquelle l'ensemble poignée est activé ; et le corps de l'ensemble poignée supportant un contact (650) et l'arbre du mécanisme de sécurité à bascule comportant un bras, dans la position inactive du mécanisme de sécurité à bascule, le bras étant espacé du contact et le contact étant dans une position ouverte, et dans la position active du mécanisme de sécurité à bascule, le bras étant en prise avec le contact et le contact étant dans une position fermée.

8. Ensemble poignée selon la revendication 7, le mécanisme de sécurité à bascule comportant un coulisseau (608) monté sur l'arbre, le coulisseau comportant un élément de butée (628) ; et l'ensemble d'entraînement comportant un élément d'accouplement (384) qui couple la vis d'entraînement à la tige d'entraînement, la vis d'entraînement, l'élément d'accouplement et la tige d'entraînement étant mobiles entre des positions rétractée et avancée à l'intérieur du corps en réponse à l'actionnement du moteur lorsque l'embrayage est dans la position de serrage/déclenchement.

9. Ensemble poignée selon la revendication 8, l'élément d'accouplement étant positionné de manière à faire obstacle au mouvement du mécanisme de sécurité à bascule de la position inactive à la position active lorsque l'élément d'accouplement est dans sa position rétractée ; et le corps supportant un élément de came effilé (622) possédant une surface de came effilée (628a) et une surface de butée proximale (684), et le coulisseau comportant une saillie (660), la saillie étant mobile le long de la surface de came effilée pour déplacer le coulisseau d'une première position sur l'arbre dans laquelle le coulisseau est aligné avec l'élément de came effilé à une seconde position sur l'arbre dans laquelle le coulisseau est positionné à l'extérieur de l'élément de came effilé lorsque le mécanisme de sécurité à bascule est déplacé de la position inactive à la position active, la saillie étant alignée avec la surface de butée proximale lorsque le mécanisme de sécurité à bascule est dans la position active pour retenir le mécanisme de sécurité à bascule dans la position active.

10. Ensemble poignée selon la revendication 9, le coulisseau étant poussé vers la première position par un mécanisme de sollicitation.

11. Dispositif chirurgical comprenant :
un ensemble poignée selon la revendication 1 et
un ensemble adaptateur (314) définissant un axe longitudinal et possédant une partie proximale (324) et une partie distale (326), l'ensemble adaptateur comportant une tige d'articulation (134), la partie proximale de l'ensemble adaptateur étant couplée à l'ensemble poignée, la tige d'articulation possédant une partie proximale couplée à la vis d'articulation et une partie distale, la tige d'entraînement s'étendant à travers l'ensemble adaptateur ; et
un ensemble outil (316) couplé de manière pivotante à la partie distale de l'ensemble adaptateur autour d'un axe qui est transversal par rapport à l'axe longitudinal de l'ensemble adaptateur, la partie distale de la tige d'articulation étant couplée à l'ensemble outil pour faire pivoter l'ensemble outil entre une position non articulée dans laquelle l'ensemble outil est aligné avec l'axe longitudinal et des positions articulées dans lesquelles l'ensemble outil est désaligné par rapport à l'axe longitudinal.

12. Dispositif chirurgical selon la revendication 11, l'au moins un sélecteur d'embrayage comportant un sélecteur d'embrayage supporté de chaque côté du corps de l'ensemble poignée, les sélecteurs d'embrayage étant couplés à l'embrayage et mobiles le long du corps de l'ensemble poignée pour déplacer l'embrayage de la position de serrage/déclenchement à la position d'articulation ; et comportant en outre un mécanisme de sécurité à bascule supporté sur le corps, le mécanisme de sécurité à bascule comportant au moins un élément de bascule et un arbre couplé à l'au moins un élément de bascule, l'arbre pouvant tourner en réponse à la manipulation de l'au moins un élément de bascule pour déplacer le mécanisme de sécurité à bascule d'une position inactive dans laquelle l'ensemble poignée est désactivé à une position active dans laquelle l'ensemble poignée est activé.

13. Dispositif chirurgical selon la revendication 12, le corps de l'ensemble poignée supportant un contact et l'arbre du mécanisme de sécurité à bascule comportant un bras, dans la position inactive du mécanisme de sécurité à bascule, le bras étant espacé du contact et le contact étant dans une position ouverte, et dans la position active du mécanisme de sécurité à bascule, le bras étant en prise avec le contact et le contact étant dans une position fermée ; et le mécanisme de sécurité à bascule comportant un coulisseau monté sur l'arbre, le coulisseau comportant un élément de butée.

14. Dispositif chirurgical selon la revendication 13, l'ensemble d'entraînement comportant un élément d'accouplement qui couple la vis d'entraînement à la tige d'entraînement, la vis d'entraînement, l'élément d'accouplement et la tige d'entraînement étant mobiles entre des positions rétractée et avancée à l'intérieur du corps en réponse à l'actionnement du moteur lorsque l'embrayage est dans la position de serrage/déclenchement ; et l'élément d'accouplement étant positionné de manière à faire obstacle au mouvement du mécanisme de sécurité à bascule de la position inactive à la position active lorsque l'élément d'accouplement est dans sa position rétractée.
